(19) Europäisches Patentamt
European Patent Office
Office européen des brevets

(11) Veröffentlichungsnummer: **0 281 883 A1**

# (12) EUROPÄISCHE PATENTANMELDUNG

(21) Anmeldenummer: **88102979.7**

(22) Anmeldetag: **29.02.88**

(51) Int. Cl.⁴: **C07D 231/40**, C07D 401/12, A01N 43/56

(30) Priorität: **10.03.87 DE 3707551**

(43) Veröffentlichungstag der Anmeldung: **14.09.88 Patentblatt 88/37**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Schallner, Otto, Dr.**
**Noldeweg 22**
**D-4019 Monheim(DE)**
Erfinder: **Gehring, Reinhold, Dr.**
**Dasnöckel 49**
**D-5600 Wuppertal 11(DE)**
Erfinder: **Stetter, Jörg, Dr.**
**Gellertweg 4**
**D-5600 Wuppertal 1(DE)**
Erfinder: **Santel, Hans-Joachim, Dr.**
**Grünstrasse 9a**
**D-5090 Leverkusen 1(DE)**
Erfinder: **Schmidt, Robert, R., Dr.**
**Im Waldwinkel 110**
**D-5060 Bergisch Gladbach 2(DE)**
Erfinder: **Lürssen, Klaus, Dr.**
**August-Kierspel-Strasse 151**
**D-5060 Bergisch Gladbach 2(DE)**
Erfinder: **Strang, Harry, Dr.**
**Unterdorfstrasse 6A**
**D-4000 Düsseldorf 31(DE)**

(54) **Substituierte 1-Arylpyrazole.**

(57) Substituierte 1-Arylpyrazole der allgemeinen Formel

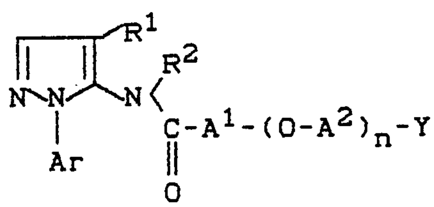

(I)

in welcher
$R^1$, $R^2$, $A^1$, $A^2$, Ar, Y und n die in der Beschreibung angegebene Bedeutung haben,
Verfahren zu deren Herstellung sowie ihre Verwendung als Herbizide und Wachstumsregulatoren.

EP 0 281 883 A1

## Substituierte 1-Arylpyrazole

Die Erfindung betrifft neue substituierte 1-Arylpyrazole, mehrere Verfahren zu ihrer Herstellung sowie ihre Verwendung als Herbizide und Pflanzenwachstumsregulatoren.

Es ist bereits bekannt, daß bestimmte 1-Arylpyrazole wie beispielsweise das 4-Cyano-5-propionamido-1-(2,4,6-trichlorphenylpyrazol) herbizide Eigenschaften besitzen (vgl. z.B. DE-OS 32 26 513).

Die herbizide Wirksamkeit dieser vorbekannten Verbindungen gegenüber Problemunkräutern ist jedoch ebenso wie ihre Verträglichkeit gegenüber wichtigen Nutzpflanzen nicht immer in allen Anwendungsgebieten völlig zufriedenstellend.

Es wurden neue substituierte 1-Arylpyrazole der allgemeinen Formel (I),

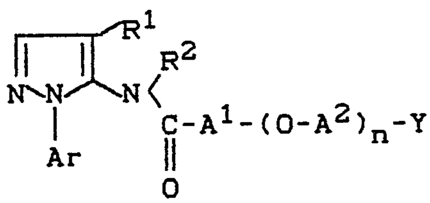

(I)

in welcher
$R^1$ für Wasserstoff oder Nitro steht,
$R^2$ für Wasserstoff, Alkyl oder für einen Rest $-\underset{\underset{O}{\|}}{C}-A^1-(O-A^2)_n-Y$ steht,
$A^1$ und $A^2$ unabhängig voneinander jeweils für einen zweifach verknüpften Alkylenrest stehen,
Y für Halogen, Hydroxy, für gegebenenfalls substituiertes Alkoxy, für gegebenenfalls substituiertes Aryloxy oder für einen Rest $-O-\underset{\underset{O}{\|}}{C}-R^3$ steht,
$R^3$ für Wasserstoff, Alkyl, Alkoxy, Amino, Alkylamino, Dialkylamino oder für jeweils gegebenenfalls substituiertes Aryl, Aryloxy oder Arylamino steht,
Ar für jeweils gegebenenfalls substituiertes Phenyl oder Pyridyl steht und
n für eine Zahl 1, 2, 3 oder 4 steht,
gefunden.

Weiterhin wurde gefunden, daß man die neuen substituierten 1-Arylpyrazole der allgemeinen Formel (I),

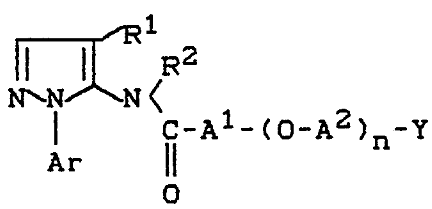

(I)

in welcher
$R^1$ für Wasserstoff oder Nitro steht,
$R^2$ für Wasserstoff, Alkyl oder für einen Rest $-\underset{\underset{O}{\|}}{C}-A^1-(O-A^2)_n-Y$ steht,
$A^1$ und $A^2$ unabhängig voneinander jeweils für einen zweifach verknüpften Alkylenrest stehen,
Y für Halogen, Hydroxy, für gegebenenfalls substituiertes Alkoxy, für gegebenenfalls substituiertes Aryloxy oder für einen Rest $-O-\underset{\underset{O}{\|}}{C}-R^3$ steht,
$R^3$ für Wasserstoff, Alkyl, Alkoxy, Amino, Alkylamino, Dialkylamino oder für jeweils gegebenenfalls substituiertes Aryl, Aryloxy oder Arylamino steht,
Ar für jeweils gegebenenfalls substituiertes Phenyl oder Pyridyl steht und
n für eine Zahl 1, 2, 3 oder 4 steht,

nach einem der im folgenden beschriebenen Verfahren erhält:

(a) Man erhält substituierte 1-Arylpyrazole der Formel (Ia),

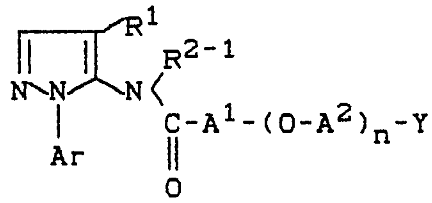

(Ia)

in welcher

$R^1$, $A^1$, $A^2$, Y, Ar und n die oben angegebene Bedeutung haben und

$R^{2-1}$ für Wasserstoff oder für einen Rest $-\underset{\underset{O}{\|}}{C}-A^2-(O-A^1)_n-Y$ steht,

wenn man 5-Amino-1-aryl-pyrazole der Formel (II),

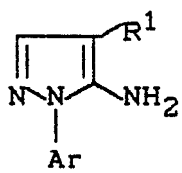

(II)

in welcher

$R^1$ und Ar die oben angegebene Bedeutung haben,

mit Acylierungsmitteln der Formel (III),

$Hal^1-\underset{\underset{O}{\|}}{C}-A^1-(O-A^2)_n-Y$ (III)

in welcher

$Hal^1$ für Halogen steht und

$A^1$, $A^2$, Y und n die oben angegebene Bedeutung haben,

gegebenenfalls in Gegenwart eines Verdünnungsmittels, gegebenenfalls in Gegenwart eines Säurebindemittels und gegebenenfalls in Gegenwart eines Katalysators umsetzt;

(b) man erhält substituierte 1-Arylpyrazole der Formel (Ib),

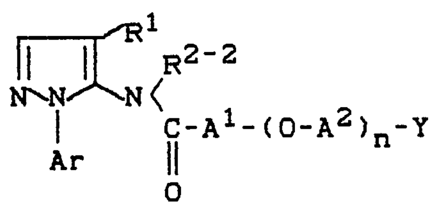

(Ib)

in welcher

$R^{2-2}$ für Alkyl steht und

$R^1$, $A^1$, $A^2$, Y, Ar und n die oben angegebene Bedeutung haben,

wenn man substituierte 1-Arylpyrazole der Formel (Ic),

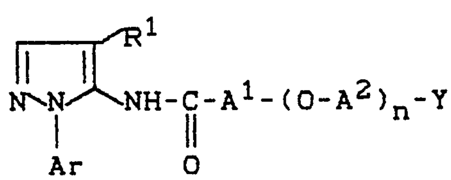

(Ic)

in welcher

$R^1$, $A^1$, $A^2$, Y, Ar und n die oben angegebene Bedeutung haben,

mit Alkylierungsmitteln der Formel (IV),

$R^{2-2}$-E (IV)

in welcher

$R^{2-2}$ für Alkyl steht und

E für eine elektronenanziehende Abgangsgruppe steht,

gegebenenfalls in Gegenwart eines Verdünnungsmittels und gegebenenfalls in Gegenwart eines Säurebindemittels sowie gegebenenfalls in Gegenwart eines Katalysators umsetzt;

(c) man erhält 4-Nitro-1-aryl-pyrazole der Formel (Id),

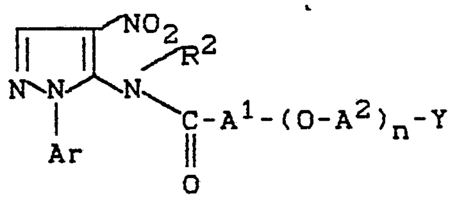

(Id)

in welcher

$R^2$, $A^1$, $A^2$, Y, Ar und n die oben angegebene Bedeutung haben,

wenn man substituierte 1-Arylpyrazole der Formel (Ie),

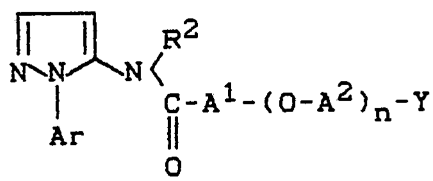

(Ie)

in welcher

$R^2$, $A^1$, $A^2$, Y, Ar und n die oben angegebene Bedeutung haben,

mit einem Nitrierungsmittel gegebenenfalls in Gegenwart eines Verdünnungsmittels und gegebenenfalls in Gegenwart eines Katalysators oder Reaktionshilfmittels umsetzt;

(d) man erhält substituierte 1-Arylpyrazole der Formel (If),

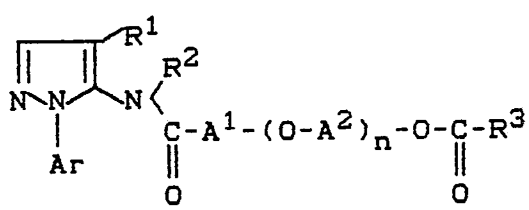

(If)

in welcher

$R^1$, $R^2$, $R^3$, $A^1$, $A^2$, Ar und n die oben angegebene Bedeutung haben,

wenn man 1-Arylpyrazole der Formel (Ig),

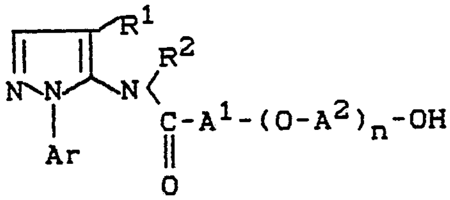

(Ig)

in welcher

$R^1$, $R^2$, $A^1$, $A^2$, Ar und n die oben angegebene Bedeutung haben,

mit Acylierungsmitteln der Formel (IV),

$$R^3-\underset{\underset{O}{\|}}{C}-Hal^2 \quad (VI)$$

in welcher

$Hal^2$ für Halogen steht und

$R^3$ die oben angegebene Bedeutung hat,

gegebenenfalls in Gegenwart eines Verdünnungsmittels, gegebenenfalls in Gegenwart eines Säurebindemittels und gegebenenfalls in Gegenwart eines Acylierungskatalysators umsetzt.

Schließlich wurde gefunden, daß die neuen substituierten 1-Arylpyrazole der allgemeinen Formel (I) herbizide, insbesondere auch selektiv-herbizide und pflanzenwachstumsregulatorische Eigenschaften besitzen.

Überraschenderweise zeigen die erfindungsgemäßen substituierten 1-Arylpyrazole der allgemeinen Formel (I) neben einer erheblich verbesserten allgemein-herbiziden Wirksamkeit gegen schwer bekämpfbare Problemunkräuter eine deutlich verbesserte Verträglichkeit gegenüber wich tigen Kulturpflanzen im Vergleich zu den aus dem Stand der Technik bekannten 1-Arylpyrazolen, wie beispielsweise das 4-Cyano-5-propionamido-1-(2,4,6-trichlorphenyl)-pyrazol, welche chemisch und wirkungsmäßig naheliegende Verbindungen sind und darüberhinaus völlig unerwartet auch pflanzenwachstumsregulatorische Eigenschaften.

Die erfindungsgemäßen substituierten 1-Arylpyrazole sind durch die Formel (I) allgemein definiert. Bevorzugt sind Verbindungen der Formel (I), bei welchen

$R^1$ für Wasserstoff oder Nitro steht,

$R^2$ für Wasserstoff, für geradkettiges oder verzweigtes Alkyl mit 1 bis 12 Kohlenstoffatomen oder für einen Rest $-\underset{\underset{O}{\|}}{C}-A^1-(O-A^2)_n-Y$ steht,

worin

$A^1$ und $A^2$ unabhängig voneinander jeweils für einen jeweils geradkettigen oder verzweigten, zweifach verknüpften Alkylenrest mit 1 bis 6 Kohlenstoffatomen stehen,

Y für Halogen, Hydroxy, für geradkettiges oder verzweigtes, gegebenenfalls einfach oder mehrfach, gleich oder verschieden durch Halogen, Cyano oder Nitro substituiertes Alkoxy mit 1 bis 6 Kohlenstaffatomen oder für gegebenenfalls einfach oder mehrfach, gleich oder verschieden substituiertes Phenyloxy steht, wobei als Substituenten infrage kommen: Halogen, Cyano, Nitro, jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 4 Kohlenstoffatomen und gegebenenfalls 1 bis 9 gleichen oder verschiedenen Halogenatomen; außerdem für einen Rest $-O-\underset{\underset{O}{\|}}{C}-R^3$ steht, wobei

$R^3$ für Wasserstoff, Amino, für jeweils geradkettiges oder verzweigtes Alkyl, Alkoxy, Alkylamino oder Dialkylamino mit jeweils 1 bis 6 Kohlenstoffatomen in den einzelnen Alkylteilen oder für jeweils gegebenenfalls einfach oder mehrfach, gleich oder verschieden substituiertes Phenyl, Phenoxy oder Phenylamino steht, wobei als Substituenten jeweils infrage kommen: Halogen, Cyano, Nitro, jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 4 Kohlenstoffatomen und gegebenenfalls 1 bis 9 gleichen oder verschiedenen Halogenatomen,

Ar für jeweils gegebenenfalls einfach oder mehrfach gleich oder verschieden substituiertes Phenyl, 2-Pyridyl, 3-Pyridyl oder 4-Pyridyl steht, wobei als Substituenten jeweils infrage kommen: Halogen, Cyano, Nitro, jeweils geradkettiges oder verzweigtes Alkyl, Alkoxy oder Alkoxycarbonyl mit jeweils 1 bis 4 Kohlenstoffatomen in den Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m-R^4$,

wobei

$R^4$ für Amino, für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht,

m für eine Zahl 0, 1 oder 2 steht und

n für eine Zahl 1, 2, 3 oder 4 steht.

Besonders bevorzugt sind substituierte 1-Arylpyrazole der allgemeinen Formel (I), bei welchen

$R^1$ für Wasserstoff oder Nitro steht,

$R^2$ für Wasserstoff, für geradkettiges oder verzweigtes Alkyl mit 1 bis 8 Kohlenstoffatomen oder für einen Rest $-\underset{\underset{O}{\|}}{C}-A^1-(O-A^2)-Y$ steht,

$A^1$ und $A^2$ unabhängig voneinander jeweils für einen jeweils geradkettigen oder verzweigten zweifach verknüpften Alkylenrest mit 1 bis 4 Kohlenstoffatomen stehen,
Y für Fluor, Chlor, Brom, Iod, Hydroxy, geradkettiges oder verzweigtes Alkoxy mit 1 bis 4 Kohlenstoffatomen oder für gegebenenfalls ein-bis dreifach, gleich oder verschieden substituiertes Phenyloxy steht, wobei als Substituenten infrage kommen: Fluor, Chlor, Brom, Cyano, Nitro, Methyl, Ethyl, n-oder i-Propyl, n-, i-, s-oder t-Butyl, Methoxy, Ethoxy, Methylthio, Trifluormethyl, Trifluormethoxy, Trifluormethylthio, Methoxycarbonyl oder Ethoxycarbonyl; außerdem für einen Rest $-O-\underset{\overset{\|}{O}}{C}-R^3$ steht,
$R^3$ für Wasserstoff, Amino, für jeweils geradkettiges oder verzweigtes Alkyl, Alkoxy, Alkylamino oder Dialkylamino mit jeweils 1 bis 4 Kohlenstoffatomen in den einzelnen Alkylteilen oder für jeweils gegebenenfalls ein-bis dreifach, gleich oder verschieden substituiertes Phenyl, Phenoxy oder Phenylamino steht, wobei als Substituenten jeweils infrage kommen: Fluor, Chlor, Brom, Cyano, Nitro, Methyl, Ethyl, n-oder i-Propyl, n-, i-, s-oder t-Butyl, Methoxy, Ethoxy, Methylthio, Trifluor methyl, Trifluormethoxy, Trifluormethylthio, Methoxycarbonyl oder Ethoxycarbonyl,
Ar für gegebenenfalls ein-bis fünffach, gleich oder verschieden substituiertes Phenyl oder für jeweils gegebenenfalls ein-bis vierfach, gleich oder verschieden substituiertes 2-Pyridyl, 3-Pyridyl oder 4-Pyridyl steht, wobei als Substituenten jeweils infrage kommen: Cyano, Nitro, Fluor, Chlor, Brom, Iod, Methyl, Ethyl, n-oder i-Propyl, n-, i-, s-oder t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^4$,
wobei
$R^4$ für Amino, Methylamino, Ethylamino, Dimethylamino, Diethylamino, Fluordichlormethyl, Difluormethyl, Tetrafluorethyl, Trichlorethyl, Trifluormethyl, Difluorchlormethyl, Methyl oder Ethyl steht,
m für eine Zahl 0, 1 oder 2 steht und
n für eine Zahl 1, 2, 3 oder 4 steht.

Ganz besonders bevorzugt sind Verbindungen der Formel (I), bei welchen
$R^1$ für Nitro steht,
$R^2$ für Wasserstoff, Methyl, Ethyl, n-oder i-Propyl, n-, i-, s-oder t-Butyl oder für einen Rest $-\underset{\overset{\|}{O}}{C}-A^1-(O-A^2)_n-Y$ steht,

$A^1$ und $A^2$ unabhängig voneinander jeweils für ein Brückenglied der Formel $-CH_2-$, $-CH_2-CH_2-$;

$-CH_2-CH_2-CH_2-$; 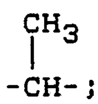 ; 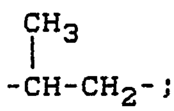 ; 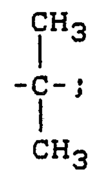 ; 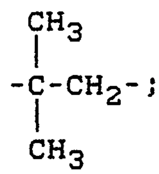 ;

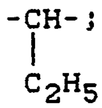 ; oder 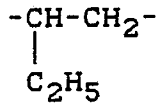 stehen,

Y für Fluor, Chlor, Brom, Methoxy, Ethoxy, n-oder i-Propoxy, n-oder i-Butoxy oder Hydroxy steht,
Ar für gegebenenfalls ein-bis fünffach, gleich oder verschieden substituiertes Phenyl oder für gegebenenfalls ein-bis vierfach gleich oder verschieden substituiertes 2-Pyridyl steht, wobei als Substituenten jeweils infrage kommen: Cyano, Nitro, Fluor, Chlor, Brom, Iod, Methyl, Ethyl, n-oder i-Propyl, n-, i-, s-oder t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^4$,
wobei

$R^4$ für Amino, Methylamino, Ethylamino, Dimethylamino, Diethylamino, Fluordichlormethyl, Difluormethyl, Tetrafluorethyl, Trichlorethyl, Trifluormethyl, Difluorchlormethyl, Methyl oder Ethyl steht,
m für eine Zahl 0, 1 oder 2 steht und
n für eine Zahl 1 oder 2 steht.

Im einzelnen seien außer den bei den Herstellungsbeispielen genannten Verbindungen die folgenden substituierten 1-Arylpyrazole der allgemeinen Formel (I) genannt:

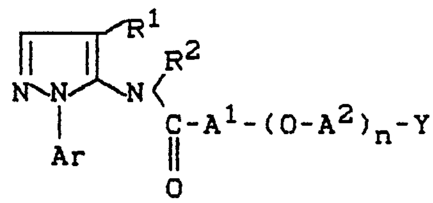 (I)

| $R^1$ | 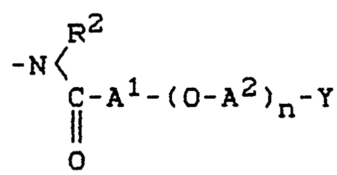 | Ar |
|---|---|---|
| $NO_2$ | $-NH-CO-CH_2-O-CH_2-CH_2-OCH_3$ | 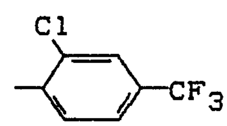 |
| $NO_2$ | $-NH-CO-CH_2-O-CH_2-CH_2-OCH_3$ | 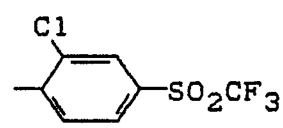 |
| $NO_2$ | $-NH-CO-CH_2-O-CH_2-CH_2-OCH_3$ | 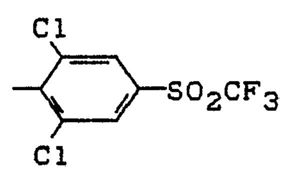 |
| $NO_2$ | $-NH-CO-CH_2-O-CH_2-CH_2-OCH_3$ | 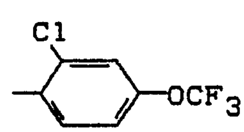 |

| $R^1$ | 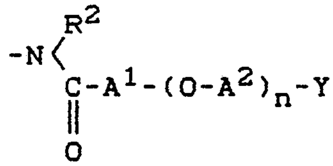 | Ar |
|---|---|---|
| $NO_2$ | $-NH-CO-CH_2-O-CH_2-CH_2-OCH_3$ | 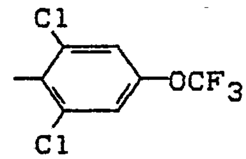 |
| $NO_2$ | $-NH-CO-CH_2-O-CH_2-CH_2-OCH_3$ | 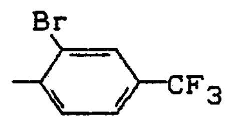 |
| $NO_2$ | $-NH-CO-CH_2-O-CH_2-CH_2-OCH_3$ | 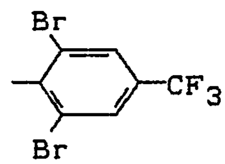 |
| $NO_2$ | $-NH-CO-CH_2-O-CH_2-CH_2-OCH_3$ | 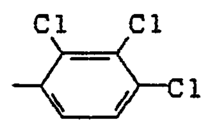 |
| $NO_2$ | $-NH-CO-CH_2-O-CH_2-CH_2-OCH_3$ | 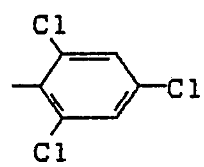 |
| $NO_2$ | $-NH-CO-CH_2-O-CH_2-CH_2-OCH_3$ | 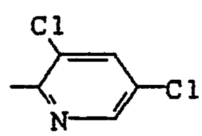 |

| $R^1$ | 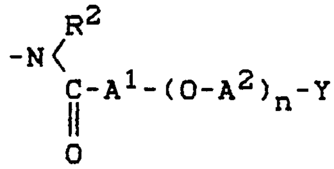 | Ar |
|---|---|---|
| $NO_2$ | $-NH-CO-CH_2-O-CH_2-CH_2-OCH_3$ | 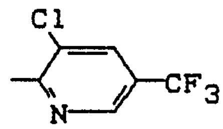 |
| $NO_2$ | $-NH-CO-CH_2-O-CH_2-CH_2-OCH_3$ | 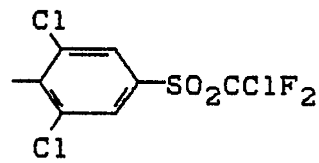 |
| $NO_2$ | $-NH-CO-CH_2-O-CH_2-CH_2-OCH_3$ | 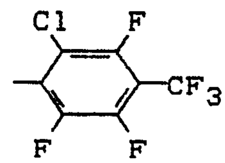 |
| $NO_2$ | $-NH-CO-CH_2-O-CH_2-CH_2-OCH_3$ | 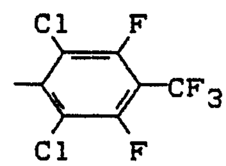 |
| $NO_2$ | $-NH-CO-CH_2-O-CH_2-CH_2-OCH_3$ | 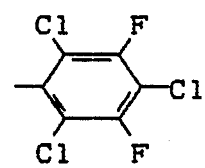 |
| $NO_2$ | $-NH-CO-CH_2-O-CH_2-CH_2-OCH_3$ | 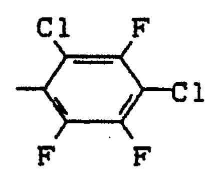 |

| $R^1$ | 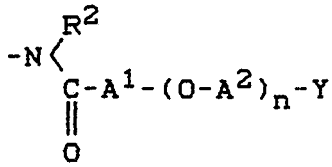 | Ar |
|---|---|---|
| $NO_2$ | $-NH-CO-CH_2-O-CH_2-CH_2-OCH_3$ | 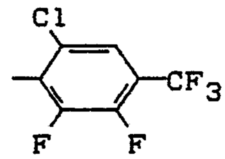 |
| $NO_2$ | $-NH-CO-CH_2-O-CH_2-CH_2-OCH_3$ | 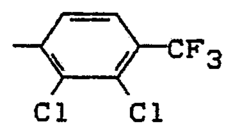 |
| $NO_2$ | $-NH-CO-CH_2-O-CH_2-CH_2-OCH_3$ | 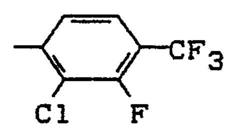 |
| $NO_2$ | $-NH-CO-CH_2-O-CH_2-CH_2-OCH_3$ | 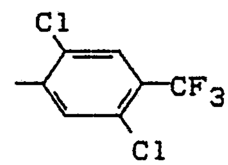 |
| $NO_2$ | $-NH-CO-CH_2-O-CH_2-CH_2-OCH_3$ | 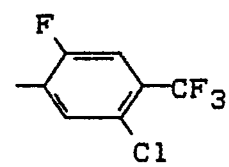 |
| $NO_2$ | $-NH-CO-CH_2-O-CH_2-CH_2-OCH_3$ | 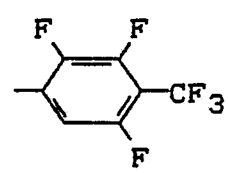 |

| $R^1$ | 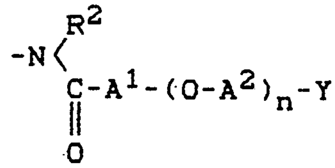 | Ar |
|---|---|---|
| $NO_2$ | $-NH-CO-CH_2-O-CH_2-CH_2-OCH_3$ | 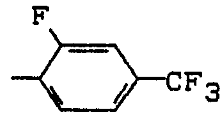 |
| $NO_2$ | $-NH-CO-CH_2-O-CH_2-CH_2-OCH_3$ | 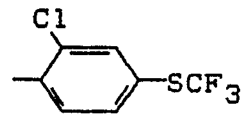 |
| $NO_2$ | $-NH-CO-CH_2-O-CH_2-CH_2-OCH_3$ | 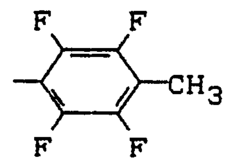 |
| $NO_2$ | $-NH-CO-CH_2-O-CH_2-CH_2-OCH_3$ | 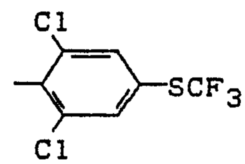 |
| $NO_2$ | $-NH-CO-CH_2-O-CH_2-CH_2-OC_2H_5$ | 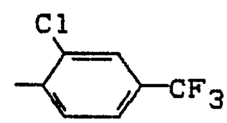 |
| $NO_2$ | $-NH-CO-CH_2-O-CH_2-CH_2-OC_2H_5$ | 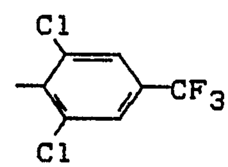 |

| $R^1$ | 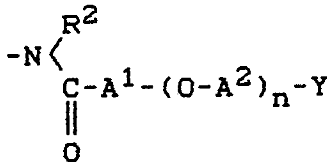 | Ar |
|---|---|---|
| $NO_2$ | $-NH-CO-CH_2-O-CH_2-CH_2-OC_2H_5$ | 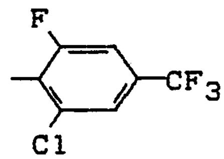 |
| $NO_2$ | $-NH-CO-CH_2-O-CH_2-CH_2-OC_2H_5$ | 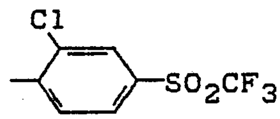 |
| $NO_2$ | $-NH-CO-CH_2-O-CH_2-CH_2-OC_2H_5$ | 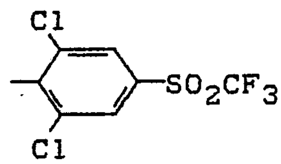 |
| $NO_2$ | $-NH-CO-CH_2-O-CH_2-CH_2-OC_2H_5$ | 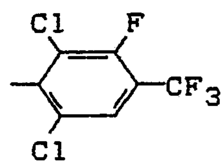 |
| $NO_2$ | $-NH-CO-CH_2-O-CH_2-CH_2-OC_2H_5$ | 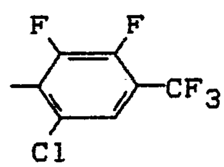 |
| $NO_2$ | $-NH-CO-CH_2-O-CH_2-CH_2-OC_2H_5$ | 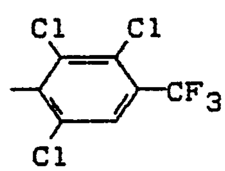 |

| $R^1$ | 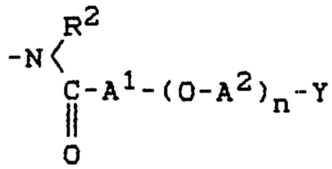 | Ar |
|---|---|---|
| $NO_2$ | $-NH-CO-CH_2-O-CH_2-CH_2-OC_2H_5$ | 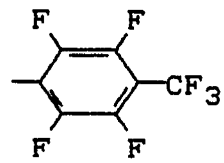 |
| $NO_2$ | $-NH-CO-CH_2-O-CH_2-CH_2-OC_2H_5$ | 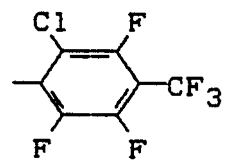 |
| $NO_2$ | $-NH-CO-CH_2-O-CH_2-CH_2-OC_2H_5$ | 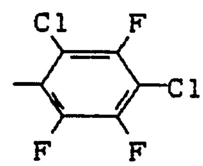 |
| $NO_2$ | $-NH-CO-CH_2-O-CH_2-CH_2-OC_2H_5$ | 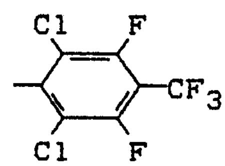 |
| $NO_2$ | $-NH-CO-CH_2-O-CH_2-CH_2-OC_2H_5$ | 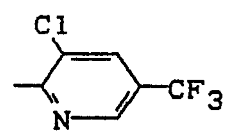 |
| $NO_2$ | $-NH-CO-CH_2-O-CH_2-CH_2-OC_2H_5$ | 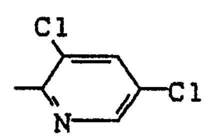 |

| $R^1$ | 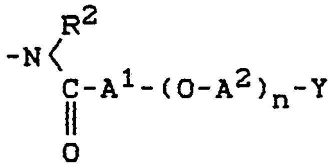 | Ar |
|---|---|---|
| $NO_2$ | $-NH-CO-CH_2-O-CH_2-CH_2-OC_2H_5$ | 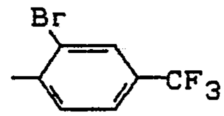 |
| $NO_2$ | $-NH-CO-CH_2-O-CH_2-CH_2-OC_2H_5$ | 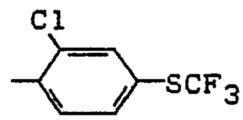 |
| $NO_2$ | $-NH-CO-CH_2-O-CH_2-CH_2-OC_2H_5$ | 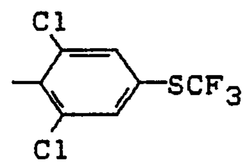 |
| $NO_2$ | $-NH-CO-CH_2-O-CH_2-CH_2-OC_2H_5$ | 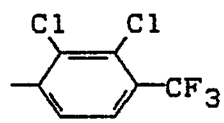 |
| $NO_2$ | $-NH-CO-CH_2-O-CH_2-CH_2-OC_2H_5$ | 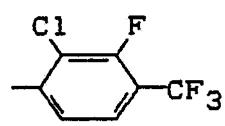 |
| $NO_2$ | $-NH-CO-CH_2-O-CH_2-CH_2-OC_2H_5$ | 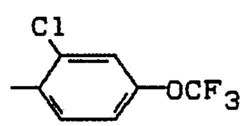 |

| $R^1$ | 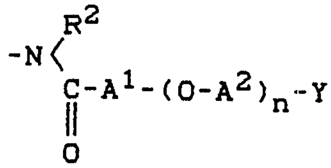 | Ar |
|---|---|---|
| $NO_2$ | $-NH-CO-CH_2-O-CH_2-CH_2-OC_2H_5$ | 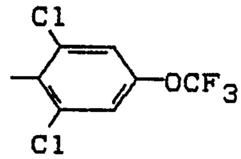 |
| $NO_2$ | $-NH-CO-CH_2-O-CH_2-CH_2-OC_2H_5$ | 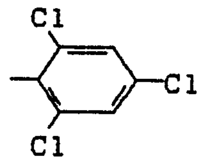 |
| $NO_2$ | $-NH-CO-CH_2-O-CH_2-CH_2-OC_2H_5$ | 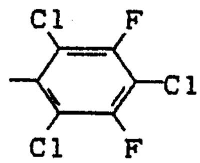 |
| $NO_2$ | $-NH-CO-CH_2-O-CH_2-CH_2-OC_2H_5$ | 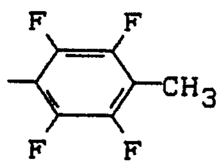 |
| $NO_2$ | $-NH-CO-CH_2-O-CH_2-CH_2-OC_2H_5$ | 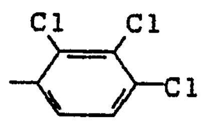 |
| $NO_2$ | $-NH-CO-CH_2-O-CH_2-CH_2-OC_2H_5$ | 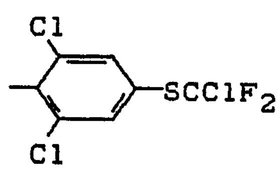 |

| $R^1$ | 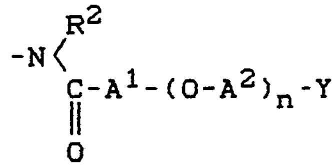 | Ar |
|---|---|---|
| $NO_2$ | $-NH-CO-CH_2-O-CH_2-CH_2-OC_4H_9-n$ | 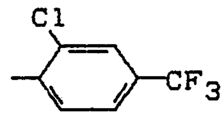 |
| $NO_2$ | $-NH-CO-CH_2-O-CH_2-CH_2-OC_4H_9-n$ | 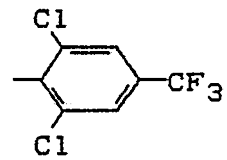 |
| $NO_2$ | $-NH-CO-CH_2-O-CH_2-CH_2-OC_4H_9-n$ | 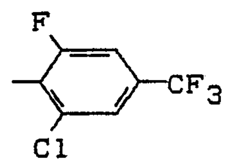 |
| $NO_2$ | $-NH-CO-CH_2-O-CH_2-CH_2-OC_4H_9-n$ | 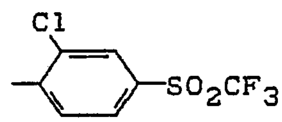 |
| $NO_2$ | $-NH-CO-CH_2-O-CH_2-CH_2-OC_4H_9-n$ | 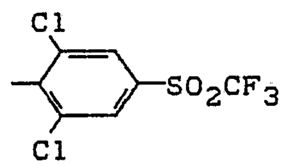 |
| $NO_2$ | $-NH-CO-CH_2-O-CH_2-CH_2-OC_4H_9-n$ | 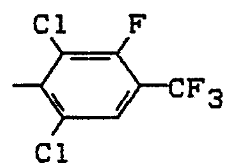 |

| $R^1$ | 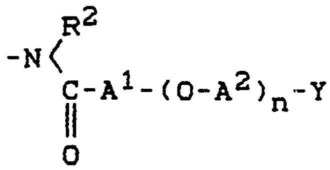 | Ar |
|---|---|---|
| $NO_2$ | $-NH-CO-CH_2-O-CH_2-CH_2-OC_4H_9-n$ | 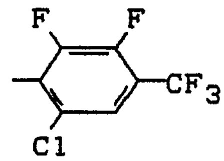 |
| $NO_2$ | $-NH-CO-CH_2-O-CH_2-CH_2-OC_4H_9-n$ | 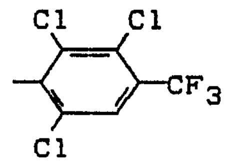 |
| $NO_2$ | $-NH-CO-CH_2-O-CH_2-CH_2-OC_4H_9-n$ | 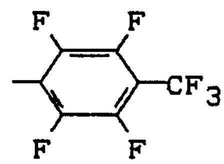 |
| $NO_2$ | $-NH-CO-CH_2-O-CH_2-CH_2-OC_4H_9-n$ | 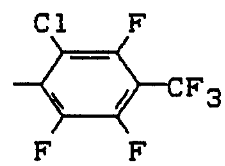 |
| $NO_2$ | $-NH-CO-CH_2-O-CH_2-CH_2-OC_4H_9-n$ | 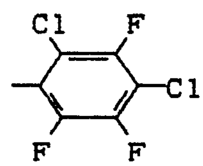 |
| $NO_2$ | $-NH-CO-CH_2-O-CH_2-CH_2-OC_4H_9-n$ | 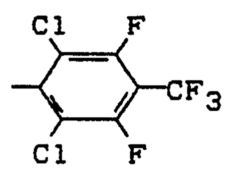 |

| $R^1$ | 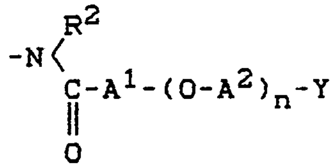 | Ar |
|---|---|---|
| $NO_2$ | $-NH-CO-CH_2-O-CH_2-CH_2-OC_4H_9-n$ | 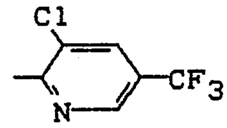 |
| $NO_2$ | $-NH-CO-CH_2-O-CH_2-CH_2-OC_4H_9-n$ | 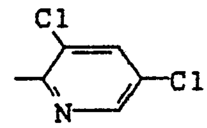 |
| $NO_2$ | $-NH-CO-CH_2-O-CH_2-CH_2-OC_4H_9-n$ | 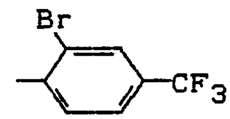 |
| $NO_2$ | $-NH-CO-CH_2-O-CH_2-CH_2-OC_4H_9-n$ | 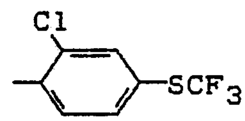 |
| $NO_2$ | $-NH-CO-CH_2-O-CH_2-CH_2-OC_4H_9-n$ | 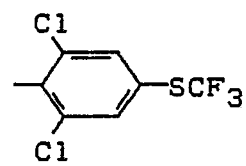 |
| $NO_2$ | $-NH-CO-CH_2-O-CH_2-CH_2-OC_4H_9-n$ | 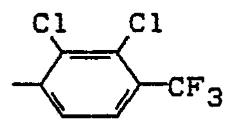 |

| $R^1$ | 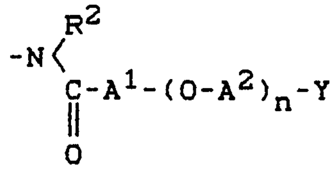 | Ar |
|---|---|---|
| $NO_2$ | $-NH-CO-CH_2-O-CH_2-CH_2-OC_4H_9-n$ | 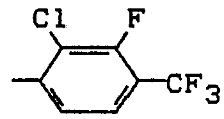 |
| $NO_2$ | $-NH-CO-CH_2-O-CH_2-CH_2-OC_4H_9-n$ | 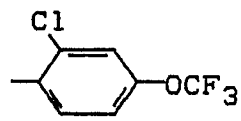 |
| $NO_2$ | $-NH-CO-CH_2-O-CH_2-CH_2-OC_4H_9-n$ | 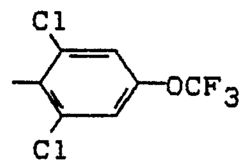 |
| $NO_2$ | $-NH-CO-CH_2-O-CH_2-CH_2-OC_4H_9-n$ | 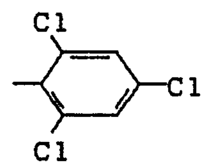 |
| $NO_2$ | $-NH-CO-CH_2-O-CH_2-CH_2-OC_4H_9-n$ | 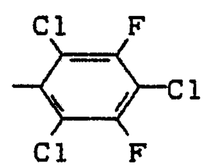 |
| $NO_2$ | $-NH-CO-CH_2-O-CH_2-CH_2-OC_4H_9-n$ | 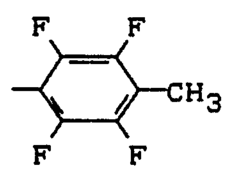 |

| $R^1$ | 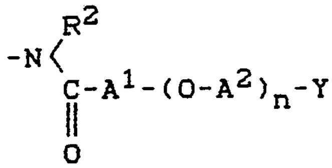 | Ar |
|---|---|---|
| $NO_2$ | $-NH-CO-CH_2-O-CH_2-CH_2-OC_4H_9-n$ | 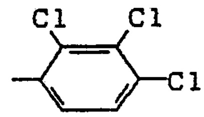 |
| $NO_2$ | $-NH-CO-CH_2-O-CH_2-CH_2-OC_4H_9-n$ | 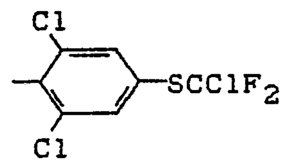 |
| $NO_2$ | $-NH-CO-CH-O-CH_2-CH_2-OCH_3$<br>$CH_3$ | 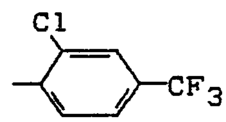 |
| $NO_2$ | $-NH-CO-CH-O-CH_2-CH_2-OCH_3$<br>$CH_3$ | 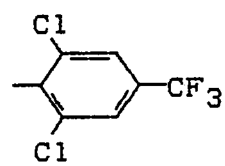 |
| $NO_2$ | $-NH-CO-CH-O-CH_2-CH_2-OCH_3$<br>$CH_3$ | 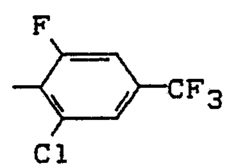 |
| $NO_2$ | $-NH-CO-CH-O-CH_2-CH_2-OCH_3$<br>$CH_3$ | 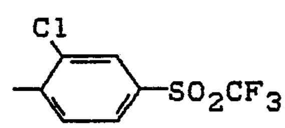 |

| | | |
|---|---|---|
| $R^1$ | 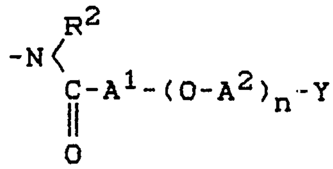 | Ar |
| $NO_2$ | 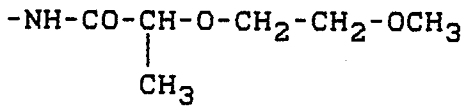 | 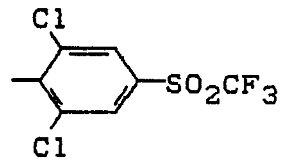 |
| $NO_2$ | 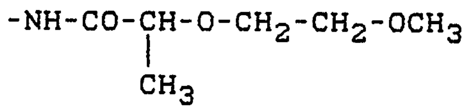 | 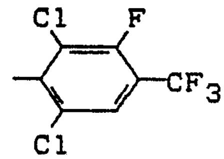 |
| $NO_2$ | 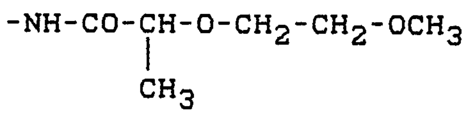 | 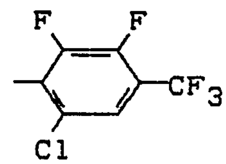 |
| $NO_2$ | 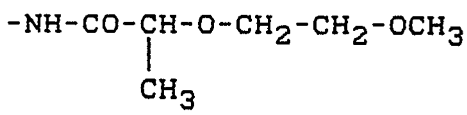 | 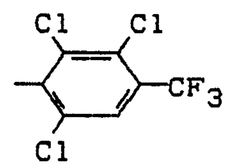 |
| $NO_2$ | 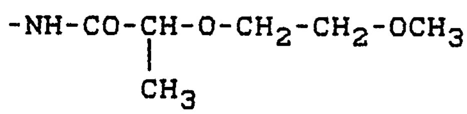 | 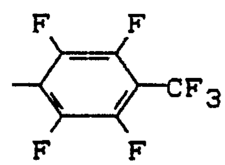 |
| $NO_2$ | 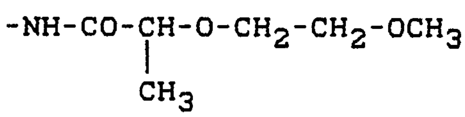 | 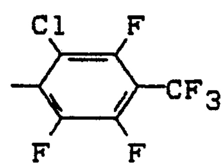 |

| $R^1$ | 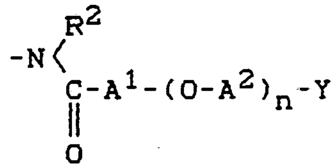 | Ar |
|---|---|---|
| $NO_2$ | 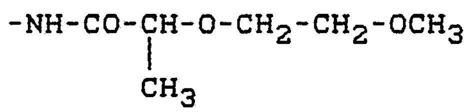 | 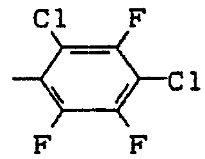 |
| $NO_2$ | 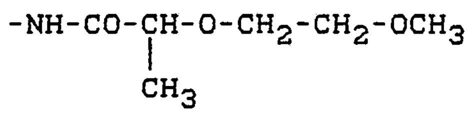 | 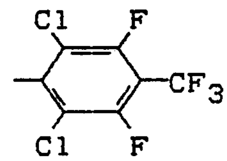 |
| $NO_2$ | 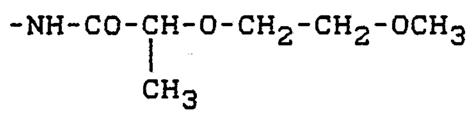 | 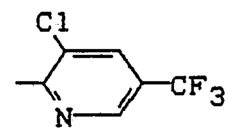 |
| $NO_2$ | 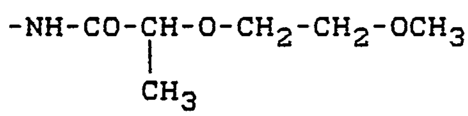 | 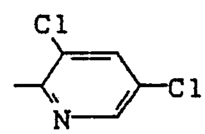 |
| $NO_2$ | 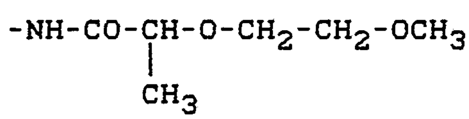 | 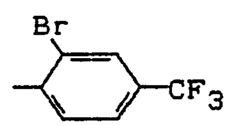 |
| $NO_2$ | 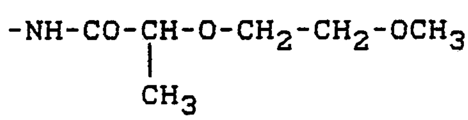 | 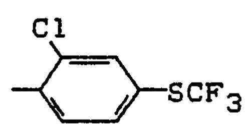 |

| $R^1$ | 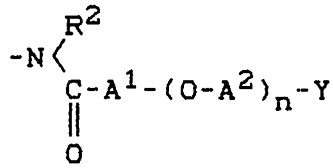 | Ar |
|---|---|---|
| $NO_2$ | 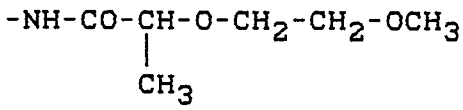 | 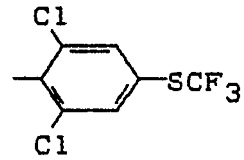 |
| $NO_2$ | 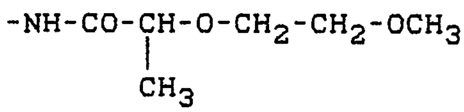 | 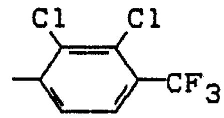 |
| $NO_2$ | 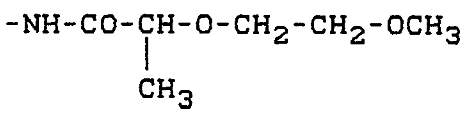 | 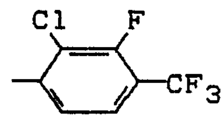 |
| $NO_2$ | 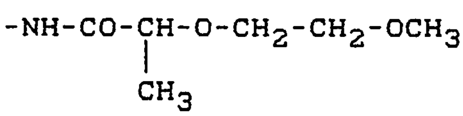 | 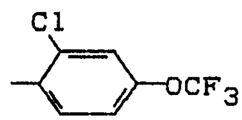 |
| $NO_2$ | 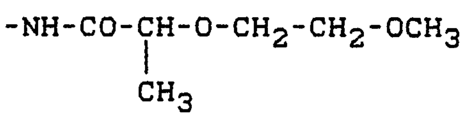 | 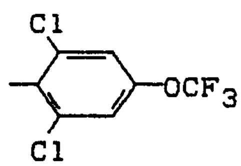 |
| $NO_2$ | 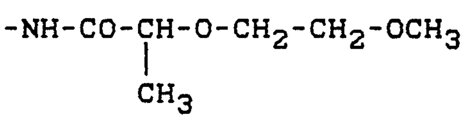 | 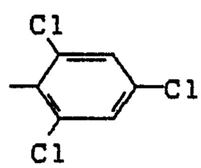 |

| $R^1$ | 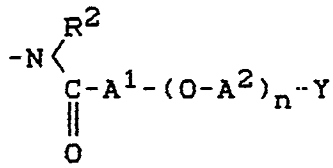 | Ar |
|---|---|---|
| $NO_2$ | $-NH-CO-CH-O-CH_2-CH_2-OCH_3$<br>$CH_3$ | 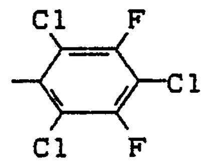 |
| $NO_2$ | $-NH-CO-CH-O-CH_2-CH_2-OCH_3$<br>$CH_3$ | 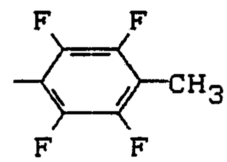 |
| $NO_2$ | $-NH-CO-CH-O-CH_2-CH_2-OCH_3$<br>$CH_3$ | 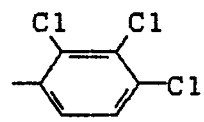 |
| $NO_2$ | $-NH-CO-CH-O-CH_2-CH_2-OCH_3$<br>$CH_3$ | 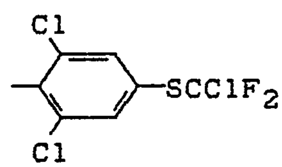 |
| $NO_2$ | $-NH-CO-CH-O-CH_2-CH_2-OC_2H_5$<br>$CH_3$ | 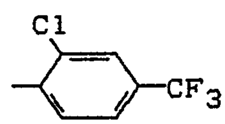 |
| $NO_2$ | $-NH-CO-CH-O-CH_2-CH_2-OC_2H_5$<br>$CH_3$ | 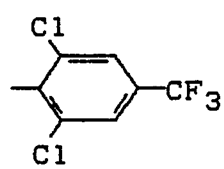 |

| $R^1$ | 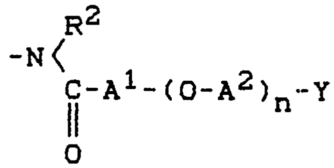 | Ar |
|---|---|---|
| $NO_2$ | $-NH-CO-CH-O-CH_2-CH_2-OC_2H_5$<br>$CH_3$ | 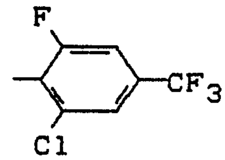 |
| $NO_2$ | $-NH-CO-CH-O-CH_2-CH_2-OC_2H_5$<br>$CH_3$ | 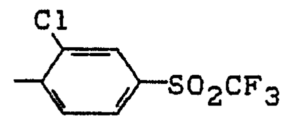 |
| $NO_2$ | $-NH-CO-CH-O-CH_2-CH_2-OC_2H_5$<br>$CH_3$ | 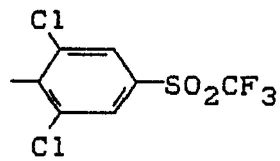 |
| $NO_2$ | $-NH-CO-CH-O-CH_2-CH_2-OC_2H_5$<br>$CH_3$ | 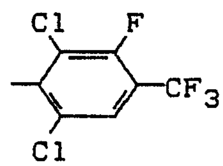 |
| $NO_2$ | $-NH-CO-CH-O-CH_2-CH_2-OC_2H_5$<br>$CH_3$ | 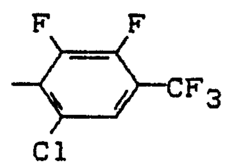 |
| $NO_2$ | $-NH-CO-CH-O-CH_2-CH_2-OC_2H_5$<br>$CH_3$ | 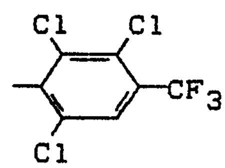 |

| $R^1$ | $-N\langle$ $R^2$<br>$C-A^1-(O-A^2)_n-Y$<br>$\|\|$<br>O | Ar |
|---|---|---|
| $NO_2$ | $-NH-CO-CH-O-CH_2-CH_2-OC_2H_5$<br>$CH_3$ | 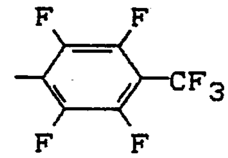 |
| $NO_2$ | $-NH-CO-CH-O-CH_2-CH_2-OC_2H_5$<br>$CH_3$ | 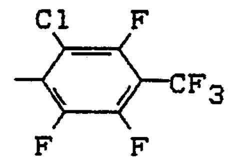 |
| $NO_2$ | $-NH-CO-CH-O-CH_2-CH_2-OC_2H_5$<br>$CH_3$ | 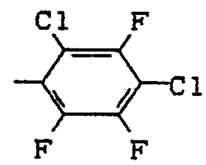 |
| $NO_2$ | $-NH-CO-CH-O-CH_2-CH_2-OC_2H_5$<br>$CH_3$ | 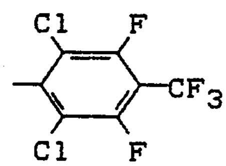 |
| $NO_2$ | $-NH-CO-CH-O-CH_2-CH_2-OC_2H_5$<br>$CH_3$ | 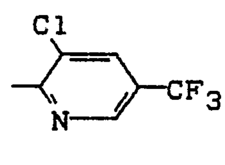 |
| $NO_2$ | $-NH-CO-CH-O-CH_2-CH_2-OC_2H_5$<br>$CH_3$ | 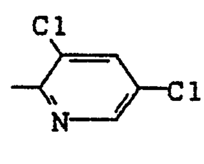 |

| $R^1$ | $-N\langle R^2$<br>$C-A^1-(O-A^2)_n-Y$<br>$\Vert$<br>O | Ar |
|---|---|---|
| $NO_2$ | $-NH-CO-CH-O-CH_2-CH_2-OC_2H_5$<br>$CH_3$ | 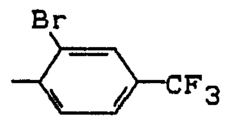 |
| $NO_2$ | $-NH-CO-CH-O-CH_2-CH_2-OC_2H_5$<br>$CH_3$ | 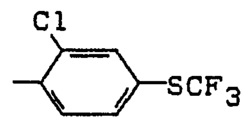 |
| $NO_2$ | $-NH-CO-CH-O-CH_2-CH_2-OC_2H_5$<br>$CH_3$ | 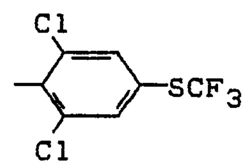 |
| $NO_2$ | $-NH-CO-CH-O-CH_2-CH_2-OC_2H_5$<br>$CH_3$ | 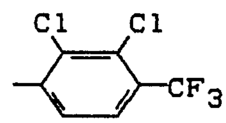 |
| $NO_2$ | $-NH-CO-CH-O-CH_2-CH_2-OC_2H_5$<br>$CH_3$ | 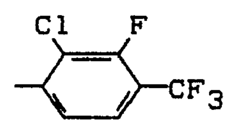 |
| $NO_2$ | $-NH-CO-CH-O-CH_2-CH_2-OC_2H_5$<br>$CH_3$ | 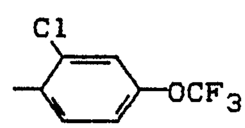 |

| $R^1$ | 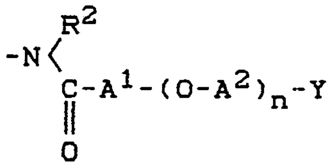 | Ar |
|---|---|---|
| $NO_2$ | $-NH-CO-CH-O-CH_2-CH_2-OC_2H_5$<br>$CH_3$ | 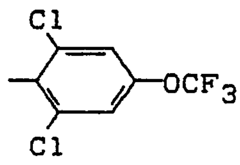 |
| $NO_2$ | $-NH-CO-CH-O-CH_2-CH_2-OC_2H_5$<br>$CH_3$ | 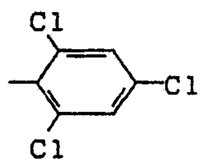 |
| $NO_2$ | $-NH-CO-CH-O-CH_2-CH_2-OC_2H_5$<br>$CH_3$ | 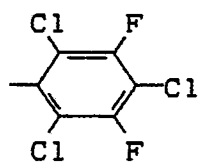 |
| $NO_2$ | $-NH-CO-CH-O-CH_2-CH_2-OC_2H_5$<br>$CH_3$ | 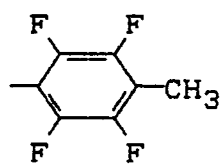 |
| $NO_2$ | $-NH-CO-CH-O-CH_2-CH_2-OC_2H_5$<br>$CH_3$ | 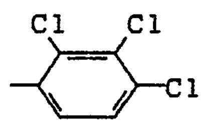 |
| $NO_2$ | $-NH-CO-CH-O-CH_2-CH_2-OC_2H_5$<br>$CH_3$ | 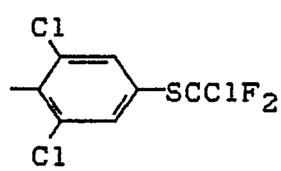 |

| $R^1$ | 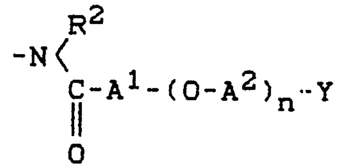 | Ar |
|---|---|---|
| $NO_2$ | 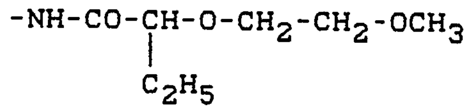 | 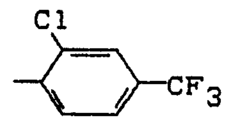 |
| $NO_2$ | 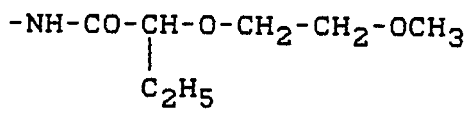 | 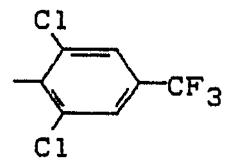 |
| $NO_2$ | 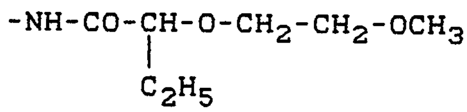 | 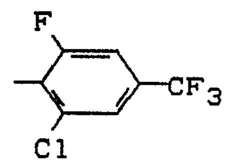 |
| $NO_2$ | 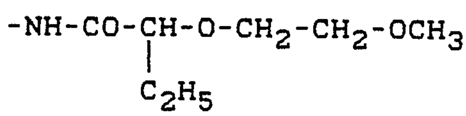 | 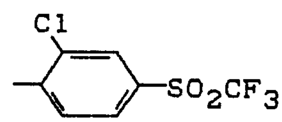 |
| $NO_2$ | 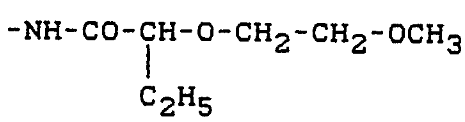 | 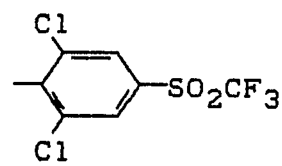 |
| $NO_2$ | 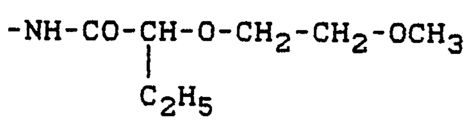 | 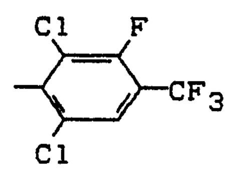 |

| $R^1$ | 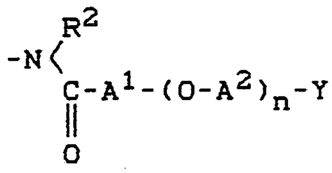 | Ar |
|---|---|---|
| $NO_2$ | 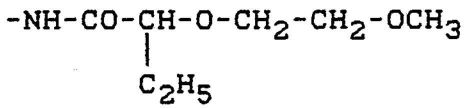 | 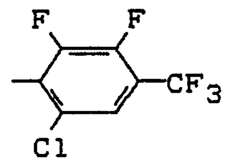 |
| $NO_2$ | 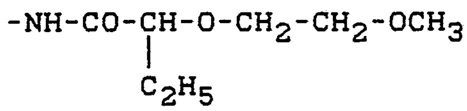 | 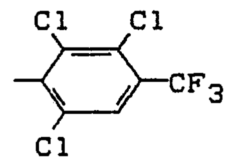 |
| $NO_2$ | 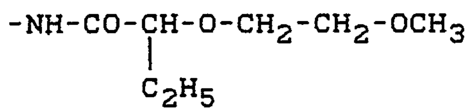 | 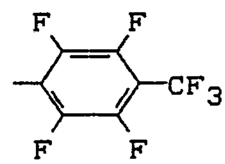 |
| $NO_2$ | 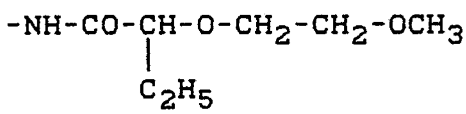 | 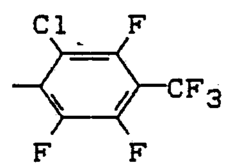 |
| $NO_2$ | 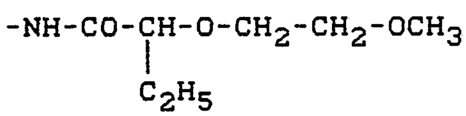 | 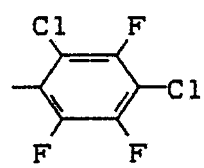 |
| $NO_2$ | 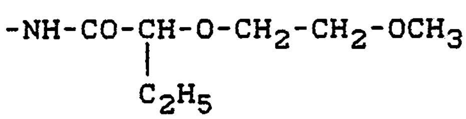 | 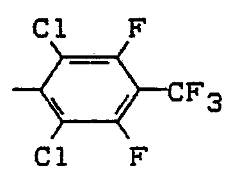 |

| $R^1$ | $-N(R^2)$<br>$C(=O)-A^1-(O-A^2)_n-Y$ | Ar |
|---|---|---|
| $NO_2$ | $-NH-CO-CH-O-CH_2-CH_2-OCH_3$<br>$C_2H_5$ | 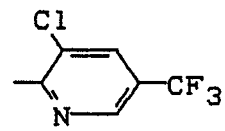 |
| $NO_2$ | $-NH-CO-CH-O-CH_2-CH_2-OCH_3$<br>$C_2H_5$ | 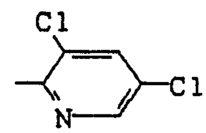 |
| $NO_2$ | $-NH-CO-CH-O-CH_2-CH_2-OCH_3$<br>$C_2H_5$ | 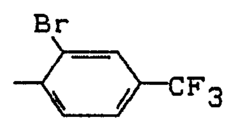 |
| $NO_2$ | $-NH-CO-CH-O-CH_2-CH_2-OCH_3$<br>$C_2H_5$ | 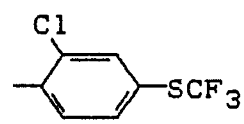 |
| $NO_2$ | $-NH-CO-CH-O-CH_2-CH_2-OCH_3$<br>$C_2H_5$ | 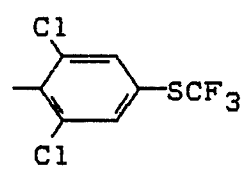 |
| $NO_2$ | $-NH-CO-CH-O-CH_2-CH_2-OCH_3$<br>$C_2H_5$ | 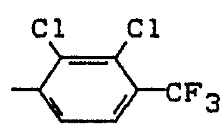 |

| $R^1$ | 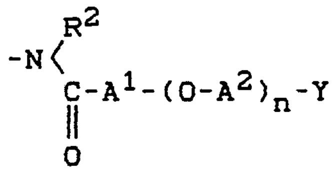 | Ar |
|---|---|---|
| $NO_2$ | $-NH-CO-CH-O-CH_2-CH_2-OCH_3$<br>$C_2H_5$ | 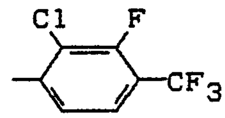 |
| $NO_2$ | $-NH-CO-CH-O-CH_2-CH_2-OCH_3$<br>$C_2H_5$ | 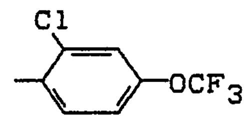 |
| $NO_2$ | $-NH-CO-CH-O-CH_2-CH_2-OCH_3$<br>$C_2H_5$ | 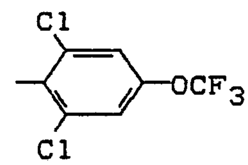 |
| $NO_2$ | $-NH-CO-CH-O-CH_2-CH_2-OCH_3$<br>$C_2H_5$ | 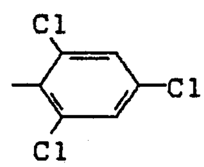 |
| $NO_2$ | $-NH-CO-CH-O-CH_2-CH_2-OCH_3$<br>$C_2H_5$ | 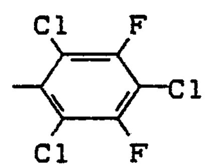 |
| $NO_2$ | $-NH-CO-CH-O-CH_2-CH_2-OCH_3$<br>$C_2H_5$ | 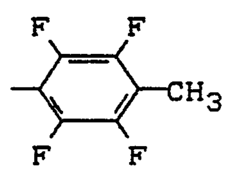 |

| $R^1$ | $-N\langle R^2$<br>$C-A^1-(O-A^2)_n-Y$<br>$O$ | Ar |
|---|---|---|
| $NO_2$ | $-NH-CO-CH-O-CH_2-CH_2-OCH_3$<br>$C_2H_5$ | 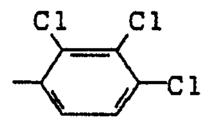 |
| $NO_2$ | $-NH-CO-CH-O-CH_2-CH_2-OCH_3$<br>$C_2H_5$ | 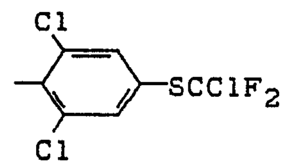 |
| $NO_2$ | $-NH-CO-CH_2-CH_2-O-CH_2-CH_2-OCH_3$ | 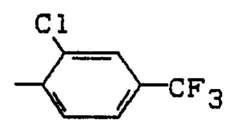 |
| $NO_2$ | $-NH-CO-CH_2-CH_2-O-CH_2-CH_2-OCH_3$ | 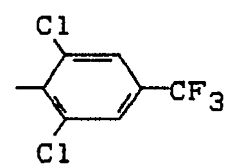 |
| $NO_2$ | $-NH-CO-CH_2-CH_2-O-CH_2-CH_2-OCH_3$ | 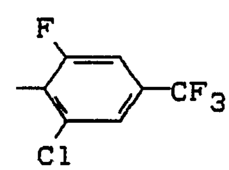 |
| $NO_2$ | $-NH-CO-CH_2-CH_2-O-CH_2-CH_2-OCH_3$ | 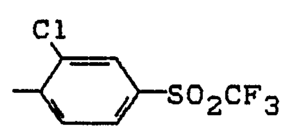 |

| $R^1$ | 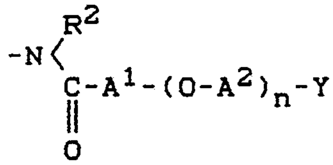 | Ar |
|---|---|---|
| $NO_2$ | $-NH-CO-CH_2-CH_2-O-CH_2-CH_2-OCH_3$ | 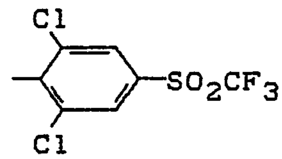 |
| $NO_2$ | $-NH-CO-CH_2-CH_2-O-CH_2-CH_2-OCH_3$ | 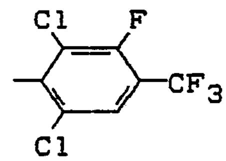 |
| $NO_2$ | $-NH-CO-CH_2-CH_2-O-CH_2-CH_2-OCH_3$ | 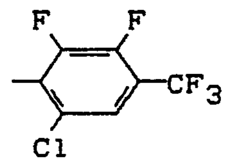 |
| $NO_2$ | $-NH-CO-CH_2-CH_2-O-CH_2-CH_2-OCH_3$ | 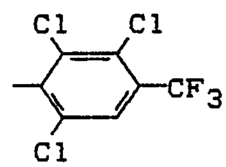 |
| $NO_2$ | $-NH-CO-CH_2-CH_2-O-CH_2-CH_2-OCH_3$ | 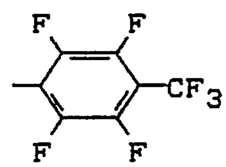 |
| $NO_2$ | $-NH-CO-CH_2-CH_2-O-CH_2-CH_2-OCH_3$ | 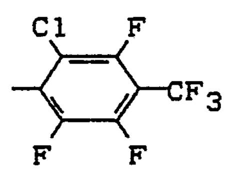 |

| $R^1$ | 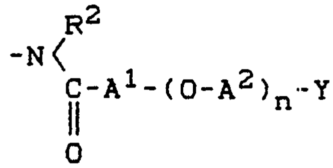 | Ar |
|---|---|---|
| $NO_2$ | $-NH-CO-CH_2-CH_2-O-CH_2-CH_2-OCH_3$ | 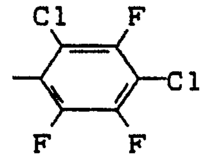 |
| $NO_2$ | $-NH-CO-CH_2-CH_2-O-CH_2-CH_2-OCH_3$ | 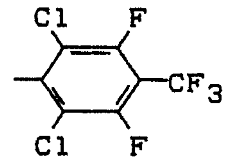 |
| $NO_2$ | $-NH-CO-CH_2-CH_2-O-CH_2-CH_2-OCH_3$ | 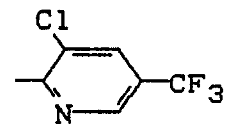 |
| $NO_2$ | $-NH-CO-CH(CH_3)-(O-CH_2-CH_2)_2-OCH_3$ | 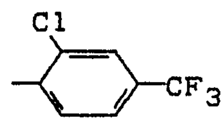 |
| $NO_2$ | $-NH-CO-CH(CH_3)-(O-CH_2-CH_2)_2-OCH_3$ | 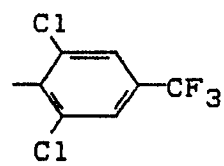 |
| $NO_2$ | $-NH-CO-CH(CH_3)-(O-CH_2-CH_2)_2-OCH_3$ | 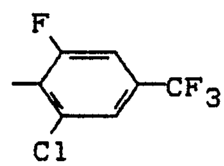 |

| $R^1$ | 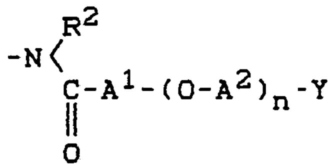 | Ar |
|---|---|---|
| $NO_2$ | $-NH-CO-CH-(O-CH_2-CH_2)_2-OCH_3$<br>$CH_3$ | 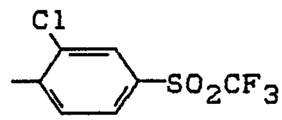 |
| $NO_2$ | $-NH-CO-CH-(O-CH_2-CH_2)_2-OCH_3$<br>$CH_3$ | 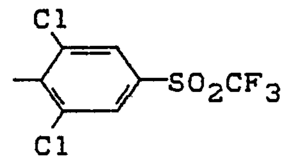 |
| $NO_2$ | $-NH-CO-CH-(O-CH_2-CH_2)_2-OCH_3$<br>$CH_3$ | 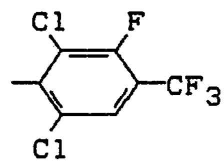 |
| $NO_2$ | $-NH-CO-CH-(O-CH_2-CH_2)_2-OCH_3$<br>$CH_3$ | 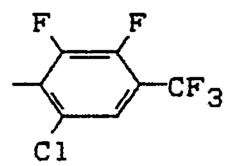 |
| $NO_2$ | $-NH-CO-CH-(O-CH_2-CH_2)_2-OCH_3$<br>$CH_3$ | 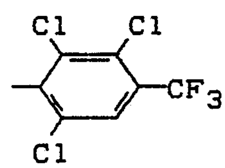 |
| $NO_2$ | $-NH-CO-CH-(O-CH_2-CH_2)_2-OCH_3$<br>$CH_3$ | 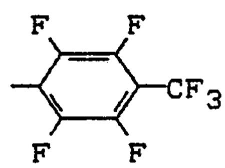 |

| $R^1$ | 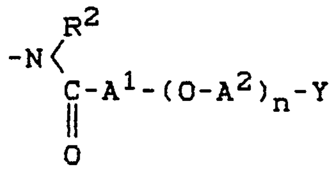 | Ar |
|---|---|---|
| $NO_2$ | $-NH-CO-\underset{\displaystyle CH_3}{\underset{\vert}{C}}H-(O-CH_2-CH_2)_2-OCH_3$ | 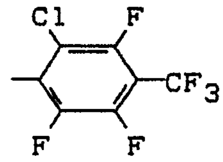 |
| $NO_2$ | $-NH-CO-\underset{\displaystyle CH_3}{\underset{\vert}{C}}H-(O-CH_2-CH_2)_2-OCH_3$ | 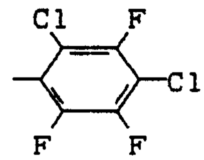 |
| $NO_2$ | $-NH-CO-\underset{\displaystyle CH_3}{\underset{\vert}{C}}H-(O-CH_2-CH_2)_2-OCH_3$ | 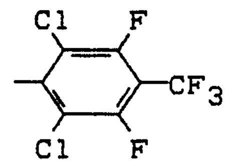 |
| $NO_2$ | $-NH-CO-\underset{\displaystyle CH_3}{\underset{\vert}{C}}H-(O-CH_2-CH_2)_2-OCH_3$ | 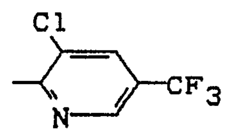 |
| $NO_2$ | $-NH-CO-CH_2-(O-CH_2-CH_2)_2-OC_2H_5$ | 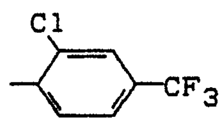 |
| $NO_2$ | $-NH-CO-CH_2-(O-CH_2-CH_2)_2-OC_2H_5$ | 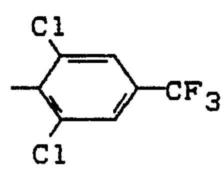 |

| $R^1$ | 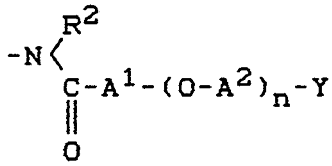 | Ar |
|---|---|---|
| $NO_2$ | $-NH-CO-CH_2-(O-CH_2-CH_2)_2-OC_2H_5$ | 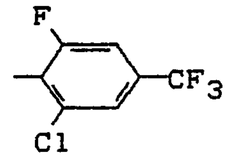 |
| $NO_2$ | $-NH-CO-CH_2-(O-CH_2-CH_2)_2-OC_2H_5$ | 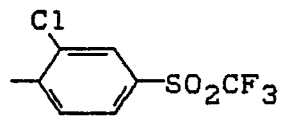 |
| $NO_2$ | $-NH-CO-CH_2-(O-CH_2-CH_2)_2-OC_2H_5$ | 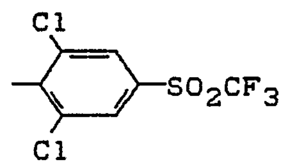 |
| $NO_2$ | $-NH-CO-CH_2-(O-CH_2-CH_2)_2-OC_2H_5$ | 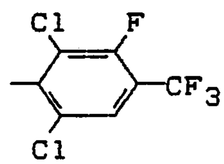 |
| $NO_2$ | $-NH-CO-CH_2-(O-CH_2-CH_2)_2-OC_2H_5$ | 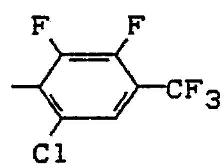 |
| $NO_2$ | $-NH-CO-CH_2-(O-CH_2-CH_2)_2-OC_2H_5$ | 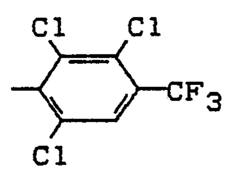 |

| $R^1$ | 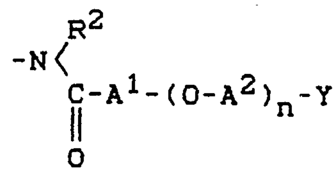 | Ar |
|---|---|---|
| $NO_2$ | $-NH-CO-CH_2-(O-CH_2-CH_2)_2-OC_2H_5$ | 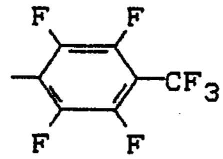 |
| $NO_2$ | $-NH-CO-CH_2-(O-CH_2-CH_2)_2-OC_2H_5$ | 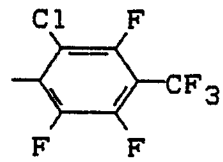 |
| $NO_2$ | $-NH-CO-CH_2-(O-CH_2-CH_2)_2-OC_2H_5$ | 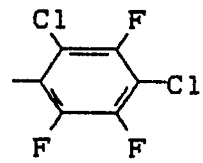 |
| $NO_2$ | $-NH-CO-CH_2-(O-CH_2-CH_2)_2-OC_2H_5$ | 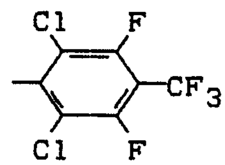 |
| $NO_2$ | $-NH-CO-CH_2-(O-CH_2-CH_2)_2-OC_2H_5$ | 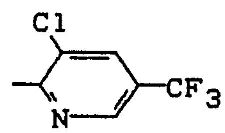 |

Verwendet man beispielweise 5-Amino-4-nitro-1-(2,6-dichlor-4-trifluormethylphenyl)-pyrazol und 2-Methoxyethoxy-acetylchlorid als Ausgangsstoffe, so läßt sich der Reaktionsablauf des erfindungsgemäßen Verfahrens (a) durch das folgende Formelschema darstellen:

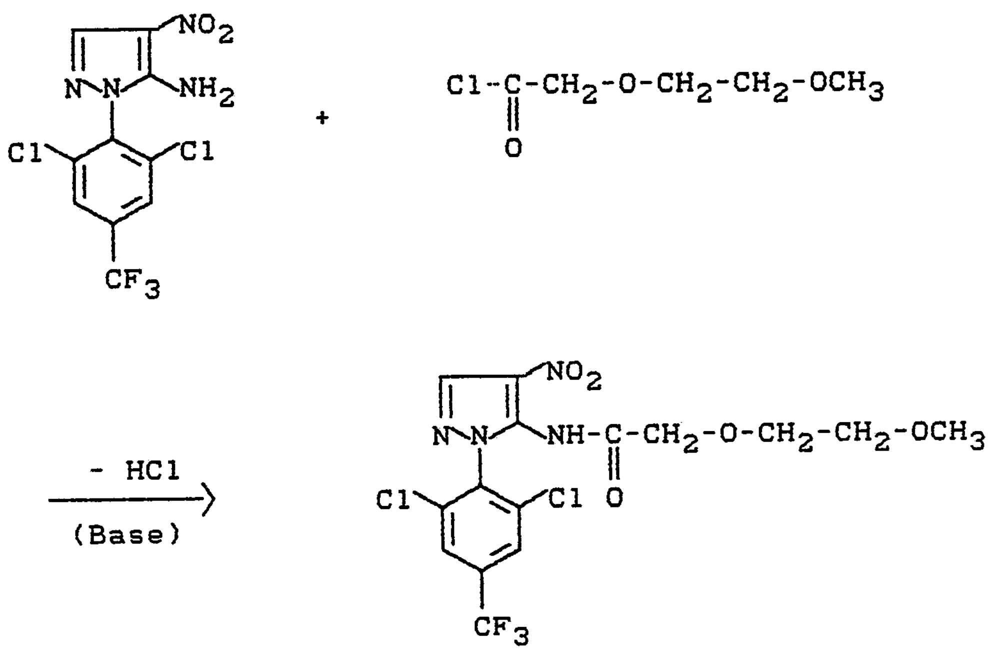


Verwendet man beispielsweise 5-(2-Methoxyethoxyacetamido)-4-nitro-1-(2,4-6-trichlorphenyl)-pyrazol und Methyliodid als Ausgangsstoffe, so läßt sich der Reaktionsablauf des erfindungsgemäßen Verfahren (b) durch das folgende Formelschema darstellen:

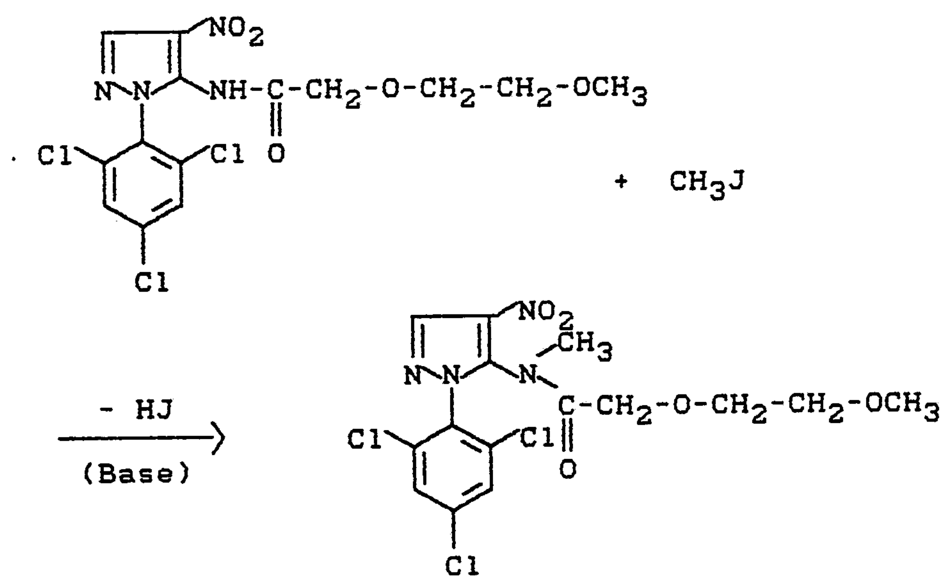


Verwendet man beispielsweise 5-(2-Ethoxyethoxyacetamido)-1-pentafluorphenyl-pyrazol als Ausgangsverbindung, so läßt sich der Reaktionsablauf des erfindungsgemäßen Verfahrens (c) durch das folgende Formelschema darstellen:

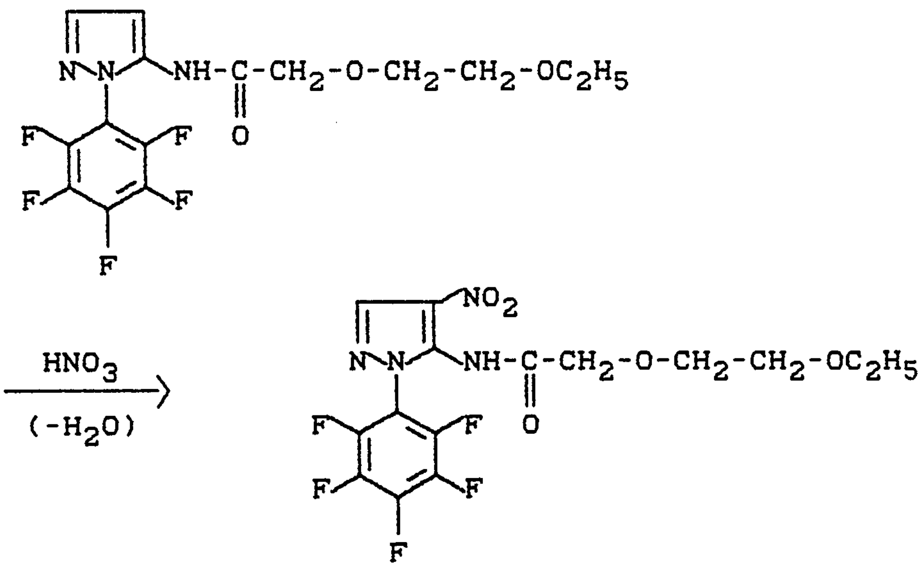


Verwendet man beispielsweise 5-(2-Hydroxyethoxyacetamido)-4-nitro-1-(2,3,6-trichlor-4-trifluormethylphenyl)-pyrazol und Propionylchlorid als Ausgangsstoffe, so läßt sich der Reaktionsablauf des erfindungsgemäßen Verfahrens (d) durch das folgende Formelschema darstellen:

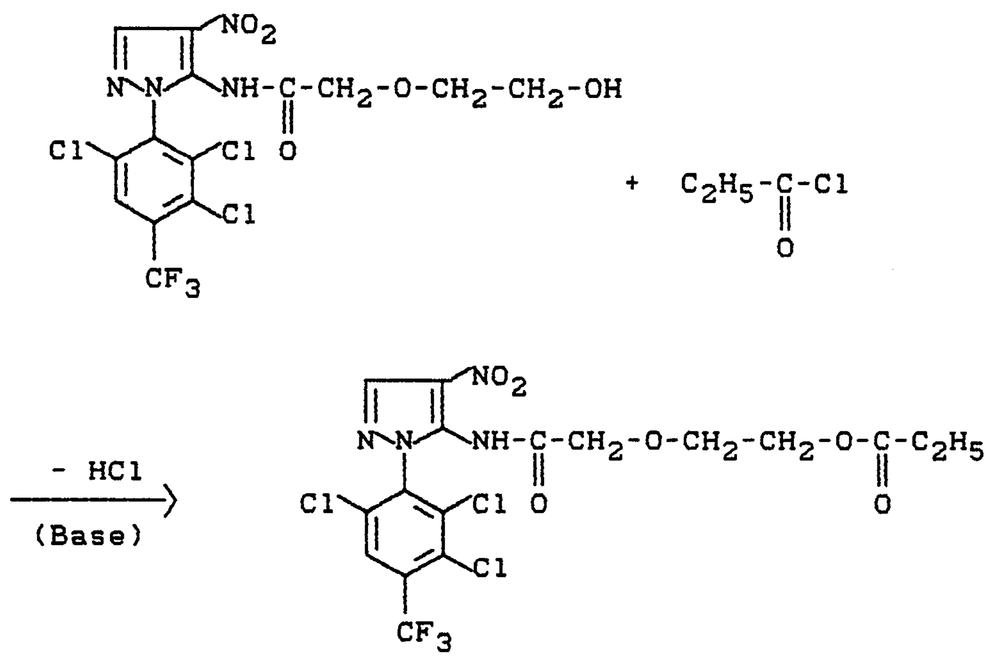


Die zur Durchführung des erfindungsgemäßen Verfahrens (a) als Ausgangsstoffe benötigten 5-Amino-1-arylpyrazole sind durch die Formel (II) allgemein definiert. In dieser Formel (II) stehen $R^1$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-aryl-pyrazole der Formel (II) sind bekannt (vgl. DE-OS 34 02 308, DE-OS 35 20 330).

Die zur Durchführung des erfindungsgemäßen Verfahrens (a) weiterhin als Ausgangsstoffe benötigten Acylierungsmittel sind durch die Formel (III) allgemein definiert. In dieser Formel (III) stehen $A^1$, $A^2$, Y und n vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden. $Hal^1$ steht vorzugsweise für Chlor oder Brom.

Die Acylierungsmittel der Formel (III) sind bekannt (vgl. z.B. Nature 160, 149-181 [1947]; DE-OS 12 00 279; Liebigs Ann. Chem. 1980, 858-862; US 4 182 711; Neth. Appl. NL 65 / 10 111 vom 06.02.1967; French Appl. M FR 5509 vom 11.12.1967; Neth. NL 6 514 035 vom 05.05.1966) oder lassen sich in Analogie zu

bekannten Verfahren nach allgemein üblichen Methoden herstellen.

Die zur Durchführung des erfindungsgemäßen Verfahrens (b) als Ausgangsstoffe benötigten substituierten 1-Arylpyrazole sind durch die Formel (Ic) allgemein definiert. In dieser Formel (Ic) stehen $R^1$, $A^1$, $A^2$, Y, Ar und n vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden.

Die substituierten 1-Arylpyrazole der Formel (Ic) sind erfindungsgemäße Verbindungen und erhältlich mit Hilfe der erfindungsgemäßen Verfahren (a), (c) oder (d).

Die zur Durchführung des erfindungsgemäßen Verfahrens (b) weiterhin als Ausgangsstoffe benötigten Alkylierungsmittel sind durch die Formel (IV) allgemein definiert. In dieser Formel (IV) steht $R^{2-1}$ vorzugsweise für geradkettiges oder verzweigtes Alkyl mit 1 bis 12, insbesondere mit 1 bis 8 Kohlenstoffatomen.

E steht vorzugsweise für Halogen, insbesondere für Chlor, Brom oder Iod oder für jeweils gegebenenfalls substituiertes Alkylsulfonyloxy, Alkoxysulfonyloxy oder Arylsulfonyloxy, wie beispielsweise Methansulfonyloxy, Methoxysulfonyloxy oder p-Toluolsulfonyloxy.

Die Alkylierungsmittel der Formel (IV) sind allgemein bekannte Verbindungen der organischen Chemie.

Die zur Durchführung des erfindungsgemäßen Verfahrens (c) als Ausgangsstoffe benötigten substituierten 1-Arylpyrazole sind durch die Formel (Ie) allgemein definiert. In dieser Formel (Ie) stehen $R^2$, $A^1$, $A^2$, Y, Ar und n vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden.

Die substituierten 1-Arylpyrazole der Formel (Ie) sind erfindungsgemäße Verbindungen und erhältlich mit Hilfe der erfindungsgemäßen Verfahren (a), (b) oder (d).

Die zur Durchführung des erfindungsgemäßen Verfahrens (d) als Ausgangsstoffe benötigten 1-Arylpyrazole sind durch die Formel (Ig) allgemein definiert. In dieser Formel (Ig) stehen $R^1$, $R^2$, $A^1$, $A^2$, Ar und n vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden.

Die 1-Arylpyrazole sind erfindungsgemäße Verbindungen und erhältlich mit Hilfe der erfindungsgemäßen Verfahren (a), (b) oder (c).

Die zur Durchführung des erfindungsgemäßen Verfahrens (d) weiterhin als Ausgangsstoffe benötigten Acylierungsmittel sind durch die Formel (VI) allgemein definiert. In dieser Formel (VI) steht $R^3$ vorzugsweise für diejenigen Reste, die bereits im Zusammenhang mit der Beschreibung der erfindungsgemäßen Stoffe der Formel (I) als bevorzugt für diese Substituenten genannt wurden. $Hal^2$ steht vorzugsweise für Chlor oder Brom. Die Acylierungsmittel der Formel (VI) sind allgemein bekannte Verbindungen der organischen Chemie.

Als Verdünnungsmittel zur Durchführung der erfindungsgemäßen Verfahren (a) und (d) kommen inerte organische Lösungsmittel infrage.

Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Die erfindungsgemäßen Verfahren (a) und (d) können gegebenenfalls in Gegenwart eines geeigneten Säurebindemittels durchgeführt werden.

Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die erfindungsgemäßen Verfahren (a) und (d) können gegebenenfalls in Gegenwart eines geeigneten Acylie rungskatalysators durchgeführt werden. Als solche verwendet man vorzugsweise Protonensäuren wie Schwefelsäure, Salzsäure, Phosphorsäure, Trifluoressigsäure oder Lewis-Säuren wie Aluminiumtrichlorid, Bortrifluorid oder Eisentrichlorid.

Die Reaktionstemperaturen können bei der Durchführung der erfindungsgemäßen Verfahren (a) und (d) in einem größeren Bereich variiert werden. Im allgemeinen arbeitet man bei Temperaturen zwischen - 20 °C und + 150 °C, vorzugsweise bei Temperaturen zwischen 0 °C und 100 °C.

Zur Durchführung des erfindungsgemäßen Verfahrens (a) setzt man pro Mol an 5-Amino-1-aryl-pyrazol

der Formel (II) im allgemeinen 1,0 bis 15,0 Mol, vorzugsweise 1,0 bis 1,5 Mol an Acylierungsmittel der Formel (III) und gegebenenfalls 1,0 bis 3,0 Mol, vorzugsweise 1,0 bis 2,0 Mol an Säurebindemittel sowie gegebenenfalls 0,1 bis 3,0 Mol, vorzugsweise 0,1 bis 2,0 Mol an Acylierungskatalysator ein.

Die Reaktionsdurchführung, Aufarbeitung und Isolierung der Reaktionsprodukte erfolgt nach allgemein üblichen Methoden.

Zur Durchführung des erfindungsgemäßen Verfahrens (d) setzt man pro Mol an 1-Arylpyrazol der Formel (Ig) im allgemeinen 1,0 bis 15,0 Mol, vorzugsweise 1,0 bis 5,0 Mol an Acylierungsmittel der Formel (VI) und gegebenen falls 1,0 bis 3,0 Mol, vorzugsweise 1,0 bis 2,0 Mol an Säurebindemittel sowie gegebenenfalls 0,1 bis 3,0 Mol, vorzugsweise 0,1 bis 2,0 Mol an Acylierungskatalysator ein.

Die Reaktionsdurchführung, Aufarbeitung und Isolierung der Reaktionsprodukte erfolgt nach allgemein üblichen Methoden.

Als Verdünnungsmittel zur Durchführung des erfindungsgemäßen Verfahrens (b) kommen ebenfalls inerte organische Lösungsmittel infrage. Vorzugsweise verwendet man die bei Verfahren (a) genannten organischen Lösungsmittel.

Das erfindungsgemäße Verfahren (b) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyl-dimethylammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Als Säurebindemittel zur Durchführung des erfindungsgemäßen Verfahrens (b) kommen alle üblicherweise verwend baren anorganischen und organischen Basen infrage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen -20 °C und +150 °C, vorzugsweise bei Temperaturen zwischen 0 °C und +100 °C.

Zur Durchführung des erfindungsgemäßen Verfahrens (b) setzt man pro Mol an substituiertem 1-Arylpyrazol der Formel (Ic) im allgemeinen 1.0 bis 20.0 Mol, vorzugsweise 1.0 bis 15.0 Mol an Alkylierungsmittel der Formel (IV) und gegebenenfalls 1.0 bis 3.0 Mol, vorzugsweise 1.0 bis 2.0 Mol an Säurebindemittel sowie 0.01 bis 1.0 Mol an Phasentransferkatalysator ein. Die Reaktionsdurchführung, Aufarbeitung und Isolierung der Reaktionsprodukte der Formel (Ib) erfolgt in allgemein üblicher Art und Weise.

Als Nitrierungsmittel zur Durchführung des erfindungsgemäßen Verfahrens (c) kommen alle üblichen Nitrierungsmittel infrage. Vorzugsweise verwendet man konzentrierte Salpetersäure oder Nitriersäure.

Als Verdünnungmittel zur Durchführung des Herstellungsverfahrens (c) kommen alle üblicherweise für derartige elektrophile Substitutionen verwendbaren Lösungmittel infrage. Vorzugsweise verwendet man die als Reagenzien infrage kommenden Säuren oder Gemische, wie beispielsweise Salpetersäure oder Nitriersäure gleichzeitig als Verdünnungsmittel. Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff als Verdünnungmittel infrage.

Als Katalysatoren oder Reaktionshilfsmittel zur Durchführung des Herstellungsverfahrens (c) kommen ebenfalls die für derartige Reaktionen üblichen Katalysatoren infrage; vorzugsweise verwendet man saure Katalysatoren wie beispielsweise Schwefelsäure, Eisen-III-chlorid oder andere Lewis-Säuren oder Acetanhydrid.

Die Reaktionstemperaturen können bei der Durchführung des Herstellungsverfahrens (c) in einem größeren Bereich variiert werden. Im allgemeinen arbeitet man zwischen - 50 °C und + 200 °C, vorzugsweise zwischen - 20 °C und + 150 °C.

Zur Durchführung des Herstellungsverfahrens (c) setzt man pro Mol substituiertem 1-Aryl-pyrazol der Formel (Ie) im allgemeinen 1,0 bis 10,0 Mol, vorzugsweise 1,0 bis 5,0 Mol an Nitrierungsmittel und gegebenenfalls 0,1 bis 10 Mol an Katalysator oder Reaktionshilfsmittel ein.

Die Reaktionsführung, Aufarbeitung und Isolierung der Reaktionsprodukte der Formel (Id) erfolgt in allgemein üblicher Art und Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen

Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung von mono-und dikotylen Unkräutern insbesondere in monokotylen Kulturen, wie beispielsweise Weizen oder Reis einsetzen.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit

Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether and Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Anwendung als Herbizide als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Auch Mischungen mit N,N-Dimethyl-N'-(3-chlor-4-methylphenyl)-harnstoff;
N,N-Dimethyl-N'-(4-isopropylphenyl)-harnstoff;
2-Chlor-N-{[(4-methoxy-6-methyl-1,3,5-triazin-2-yl)-amino]-carbonyl}-benzolsulfonamid;
2-{[[((4-ethoxy-6-methyl-1,3,5-triazin-2-yl)-amino)-carbonyl]-amino]-sulfonyl}-benzoesäure oder deren Methylester
4-Ethylamino-2-t-butylamino-6-methylthio-s-triazin;
4-Amino-6-t-butyl-3-ethylthio-1,2,4-triazin-5(4H)-on N-Methyl-2-(1,3-benzthiazol-2-yloxy)-acetanilid;
α-Chlor-2',6'-diethyl-N-(2-propoxyethyl)-acetanilid;
N,N-Diisopropyl-S-(2,3,3-trichlorallyl)-thiolcarbamat;
N-(1-Ethylpropyl)-3,4-dimethyl-2,6-dinitroanilin;
Methyl-5-(2,4-dichlorphenoxy)-2-nitrobenzoat;
Methyl-2-[4,5-dihydro-4-methyl-4-(1-methylethyl)-5-oxo-1H-imidazol-2-yl]-4(5)-methylbenzoat
2-[4-(2,4-Dichlorphenoxy)-phenoxy]-propionsäuremethylester;
2-[4-(3,5-Dichlorpyrid-2-yloxy)-phenoxy]-propionsäure-(trimethylsilylmethylester);
3,5-Diiod-4-hydroxybenzonitril; 3,5-Dibrom-4-hydroxybenzonitril;
[(4-Amino-3,5-dichlor-6-fluor-2-pyridinyl)-oxy]-essigsäure;
bzw. -1-methylheptylester; 2,4-Dichlorphenoxyessigsäure; 2,4-Dichlorphenoxypropionsäure; (2-Methyl-4-chlorphenoxy)-essigsäure; (4-Chlor-2-methylphenoxy)-propionsäure; N-(3,4-Dichlorphenyl)-propionamid und 3-Isopropyl-2,1,3-benzothiadiazin-4-on-2,2-dioxid sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die erfindungsgemäßen Wirkstoffe können auch bei der Anwendung als Wachstumsregulatoren in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können dabei als solche, in Form ihrer Formulierungen oder den daraus bereiteten Anwendungs formen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. die Anwendung geschieht in üblicher Weise, z. B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können bei der Anwendung als Wachstumsregulatoren ebenfalls in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

Herstellungsbeispiele:

Beispiel 1:

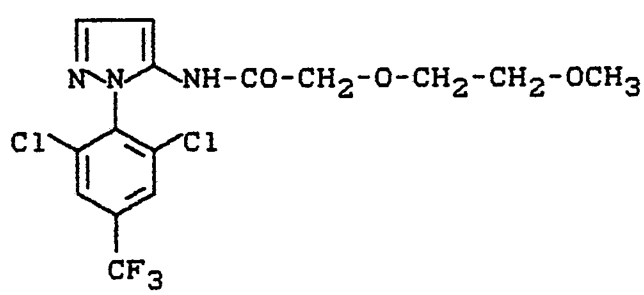

(Verfahren a)

Zu 20,5 g (0,069 Mol) 5-Amino-1-(2,6-dichlor-4-trifluormethylphenyl)-pyrazol (vgl. DE-OS 34 02 308) in 110 ml Acetonitril gibt man bei 20 °C unter Rühren nacheinander 7 ml (0,088 Mol) Pyridin und 13,3 g (0,087 Mol) 2-Methoxy-ethoxyacetylchlorid (vgl. US-PS 4 182 722) und rührt 12 Stunden bei Raumtemperatur. Zur Aufarbeitung gießt man die Mischung in 200 ml Eiswasser, filtriert das ausgefallene Produkt ab, wäscht mehrfach mit Wasser nach und trocknet im Vakuum.

Man erhält 26,6 g (93,3 % der Theorie) an 5-(2-Methoxyethoxyacetamido)-1-(2,6-dichlor-4-trifluormethylphenyl)-pyrazol vom Schmelzpunkt 158 °C - 160 °C.

Beispiel 2:

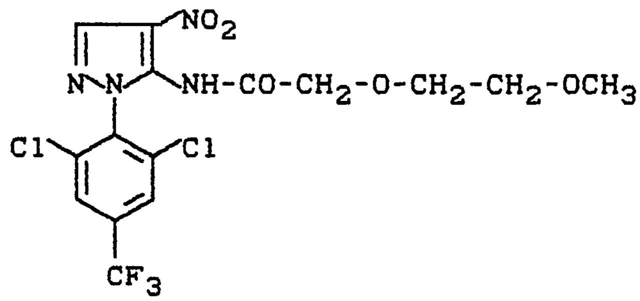

(Verfahren c)

Zu 4,1 g (0,01 Mol) 5-(2-Methoxyethoxyacetamido)-1-(2,6-dichlor-4-trifluormethylphenyl)-pyrazol in 20 ml Eisessig gibt man bei ca. 15 °C nacheinander 1,4 ml (0,015 Mol) Acetanhydrid und 0,5 ml (0,012 Mol) 98prozentige Salpetersäure. Nach beendeter Zugabe rührt man 20 Stunden bei Raumtemperatur, gießt die Mischung in 150 ml Eiswasser, filtriert das ausgefallene Produkt ab, wäscht mehrfach mit Wasser nach und trocknet im Vakuum.

Man erhält 4,2 g (92 % der Theorie) an 5-(2-Methoxyethoxyacetamido)-4-nitro-1-(2,6-dichlor-4-trifluormethylphenyl)-pyrazol vom Schmelzpunkt 103 °C -105 °C.

In entsprechender Weise und gemäß den allgemeinen Angaben zur Herstellung erhält man die folgenden substituierten 1-Arylpyrazole der allgemeinen Formel (I):

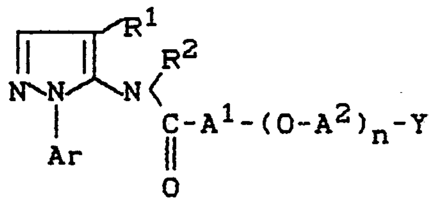

(I)

| Bsp. Nr. | $R^1$ | $R^2$ | $A^1-(O-A^2)_n-Y$ | Ar | physikalische Eigenschaften |
|---|---|---|---|---|---|
| 3 | H | H | $-CH_2-O-(CH_2)_2-OCH_3$ | 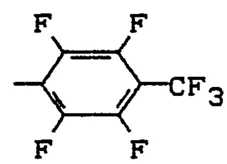 | $^1H$-NMR*): 7,8 |
| 4 | H | H | $-CH_2-O-CH_2-CH_2-OCH_3$ | 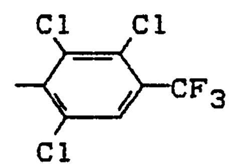 | Fp 139-142 °C |
| 5 | H | H | $-CH_2-O-CH_2-CH_2-OCH_3$ | 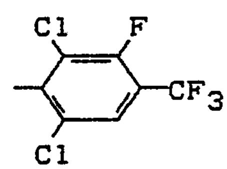 | Fp 131-133 °C |

| Bsp. Nr. | $R^1$ | $R^2$ | $A^1-(O-A^2)_n-Y$ | Ar | physikalische Eigenschaften |
|---|---|---|---|---|---|
| 6 | $NO_2$ | H | $-CH_2-O-CH_2-CH_2-OCH_3$ | 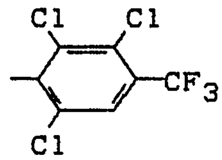 | Fp 78-86 °C |
| 7 | $NO_2$ | H | $-CH_2-O-CH_2-CH_2-OCH_3$ | 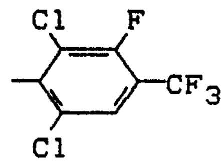 | Fp 58-61 °C |
| 8 | $NO_2$ | H | $-CH_2-O-CH_2-CH_2-OCH_3$ | 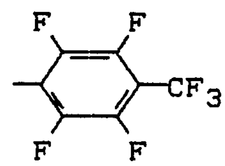 | Fp 64-66 °C |
| 9 | H | H | $-CH_2-(O-CH_2-CH_2)_2-OCH_3$ | 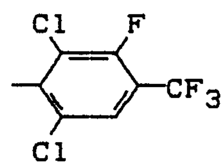 | $^1$H-NMR*): 7,8 |
| 10 | H | H | $-CH_2-(O-CH_2-CH_2)_2-OCH_3$ | 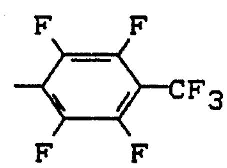 | $^1$H-NMR*): 7,82 |

| Bsp. Nr. | $R^1$ | $R^2$ | $A^1-(O-A^2)_n-Y$ | Ar | physikalische Eigenschaften |
|---|---|---|---|---|---|
| 11 | H | H | $-CH_2-(O-CH_2-CH_2)_2-OCH_3$ | 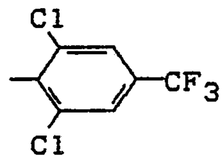 | $^1$H-NMR*): 7,8 |
| 12 | H | H | $-CH_2-(O-CH_2-CH_2)_2-OCH_3$ | 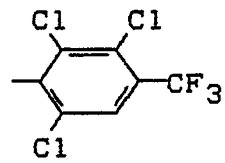 | $^1$H-NMR*); 7,78 |
| 13 | H | H | $-CH_2-(O-CH_2-CH_2)_2-OCH_3$ | 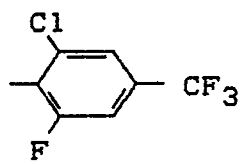 | $^1$H-NMR*): 7,78 |
| 14 | $NO_2$ | H | $-CH_2-(O-CH_2-CH_2)_2-OCH_3$ | 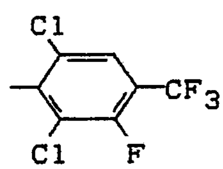 | Fp 63-66 °C |
| 15 | $NO_2$ | H | $-CH_2-(O-CH_2-CH_2)_2-OCH_3$ | 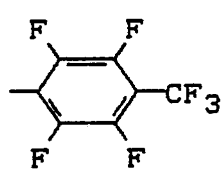 | Fp 50-53 °C |

| Bsp. Nr. | $R^1$ | $R^2$ | $A^1-(O-A^2)_n-Y$ | Ar | physikalische Eigenschaften |
|---|---|---|---|---|---|
| 16 | $NO_2$ | H | $-CH_2-(O-CH_2-CH_2)_2-OCH_3$ | 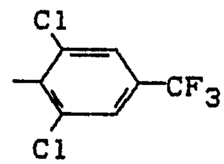 | Fp 44-47 °C |
| 17 | $NO_2$ | H | $-CH_2-(O-CH_2-CH_2)_2-OCH_3$ | 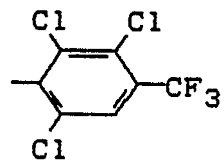 | Fp 62-64 °C |
| 18 | $NO_2$ | H | $-CH_2-(O-CH_2-CH_2)_2-OCH_3$ | 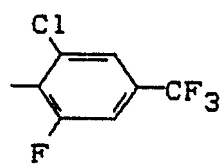 | $^1H$-NMR*): 8,38 |
| 19 | $NO_2$ | H | $-CH_2-O-CH_2-CH_2-OCH_3$ | 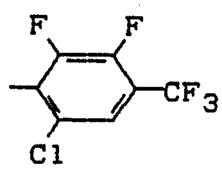 | $^1H$-NMR*): 8,36 |
| 20 | $NO_2$ | H | $-CH_2-O-CH_2-CH_2-OCH_3$ | 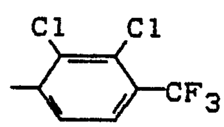 | $^1H$-NMR*): 8,36 |

| Bsp. Nr. | $R^1$ | $R^2$ | $A^1-(O-A^2)_n-Y$ | Ar | physikalische Eigenschaften |
|---|---|---|---|---|---|
| 21 | $NO_2$ | H | $-CH_2-O-CH_2-CH_2-OCH_3$ | 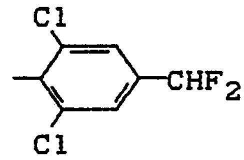 | Öl |
| 22 | $NO_2$ | H | $-CH_2-O-CH_2-CH_2-OCH_3$ | 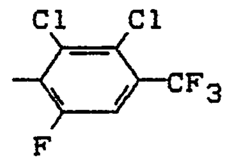 | Fp 88-89° C |
| 23 | $NO_2$ | H | $-CH_2-O-CH_2-CH_2-OCH_3$ | 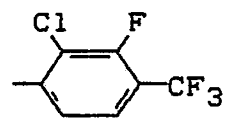 | $^1$H-NMR*): 8.34(s) |
| 24 | $NO_2$ | H | $-CH_2-O-CH_2-CH_2-OCH_3$ | 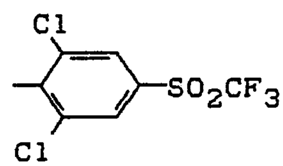 | Fp 119° -124° C |
| 25 | $NO_2$ | H | $-\overset{CH_3}{\overset{\vert}{CH}}-O-CH_2-CH_2-OCH_3$ | 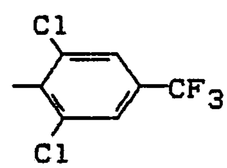 | Fp 92° -94° C |

| Bsp. Nr. | $R^1$ | $R^2$ | $A^1-(O-A^2)_n-Y$ | Ar | physikalische Eigenschaften |
|---|---|---|---|---|---|
| 26 | $NO_2$ | H | $CH_3$<br>\|<br>$-CH-O-CH_2-CH_2-OCH_3$ | 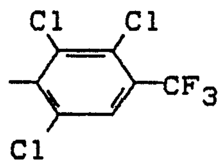 | $^1H$-NMR*):<br>8.38(s) |
| 27 | $NO_2$ | H | $CH_3$<br>\|<br>$-CH-O-CH_2-CH_2-OCH_3$ | 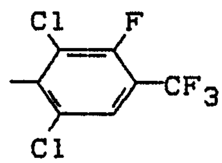 | $^1H$-NMR*):<br>8.39(s) |
| 28 | $NO_2$ | H | $CH_3$<br>\|<br>$-CH-O-CH_2-CH_2-OCH_3$ | 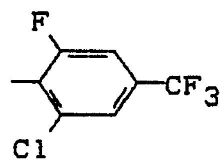 | Fp 77-79°C |
| 29 | $NO_2$ | H | $CH_3$<br>\|<br>$-CH-O-CH_2-CH_2-OCH_3$ | 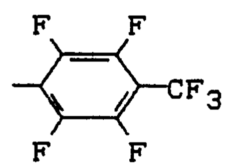 | $^1H$-NMR*):<br>8.39(s) |
| 30 | $NO_2$ | H | $CH_3$<br>\|<br>$-CH-O-CH_2-CH_2-OCH_3$ | 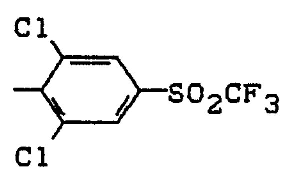 | Fp 70-79°C |

| Bsp. Nr. | $R^1$ | $R^2$ | $A^1-(O-A^2)_n-Y$ | Ar | physikalische Eigenschaften |
|---|---|---|---|---|---|
| 31 | $NO_2$ | H | $-CH(CH_3)-O-CH_2-CH_2-OCH_3$ | 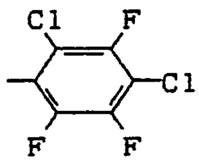 | $^1H$-NMR*): 8.37(s) |
| 32 | $NO_2$ | H | $-CH(CH_3)-O-CH_2-CH_2-OCH_3$ | 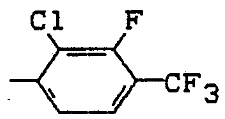 | $^1H$-NMR*) 8.36(s) |
| 33 | $NO_2$ | H | $-CH(CH_3)-O-CH_2-CH_2-OCH_3$ | 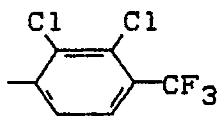 | Fp 78-86° C |
| 34 | $NO_2$ | H | $-CH(CH_3)-O-CH_2-CH_2-OCH_3$ | 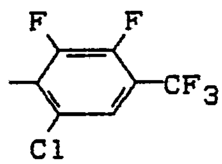 | $^1H$-NMR*) 8.39(s) |
| 35 | $NO_2$ | H | $-CH(CH_3)-O-CH_2-CH_2-OCH_3$ | 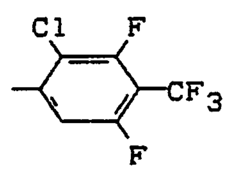 | $^1H$-NMR*): 8.33(s) |

*) Die $^1H$-NMR-Spektren wurden in $CDCl_3$ mit Tetramethylsilan (TMS) als innerem Standard aufgenommen. Angegeben ist die chemische Verschiebung des Pyrazol-3-Wasserstoffes als δ-Wert in ppm.

Anwendungsbeispiele:

In den folgenden Anwendungsbeispielen wurde die nachstehend aufgeführte Verbindung als Vergleichsubstanz eingesetzt:

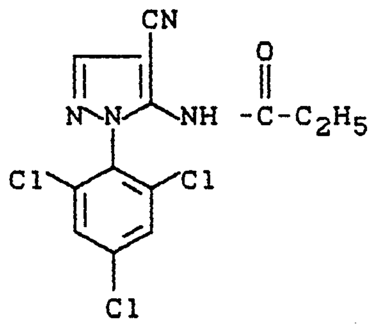

(A)

4-Cyano-5-propionamido-1-(2,4,6-trichlorphenyl)-pyrazol (bekannt aus DE-OS 3 226 513)

Beispiel A

Pre-emergence-Test Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen beispielsweise die Verbindungen gemäß den Herstellungsbeispielen 2, 6, 7 und 8 eine deutliche Überlegenheit in der Wirksamkeit gegen monokotyle und dikotyle Unkräuter gegenüber der Vergleichsverbindung (A).

Beispiel B

Post-emergence-Test Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen beispielsweise die Verbindungen gemäß den Herstellungsbeispielen 2, 6, 7 und 8 eine deutliche Überlegenheit in der Wirksamkeit ebenso wie in der Nutzpflanzenselektivität gegenüber der Vergleichssubstanz (A).

Beispiel C

Entlaubung und Austrocknung der Blätter bei BaumwolleLösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
+ leichtes Austrocknen der Blätter, geringer Blattfall
+ + starkes Austrocknen der Blätter, starker Blattfall
+ + + sehr starkes Austrocknen der Blätter, sehr starker Blattfall

In diesem Test zeigen beispielsweise die Verbindungen gemäß der Herstellungsbeispiele 2, 6, 7 und 8 eine sehr gute Wirkung.

**Ansprüche**

1. Substituierte 1-Arylpyrazole der allgemeinen Formel (I),

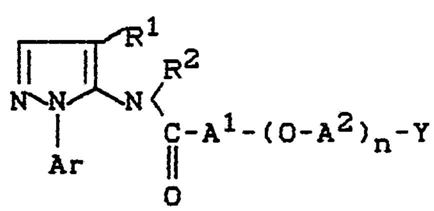

(I)

in welcher
$R^1$ für Wasserstoff oder Nitro steht,
$R^2$ für Wasserstoff, Alkyl oder für einen Rest $-\underset{\overset{\|}{O}}{C}-A^1-(O-A^2)_n-Y$ steht,
$A^1$ und $A^2$unabhängig voneinander jeweils für einen zweifach verknüpften Alkylenrest stehen,
Y für Halogen, Hydroxy, für gegebenenfalls substituiertes Alkoxy, für gegebenenfalls substituiertes Aryloxy oder für einen Rest $-O-\underset{\overset{\|}{O}}{C}-R^3$ steht,
$R^3$ für Wasserstoff, Alkyl, Alkoxy, Amino, Alkylamino, Dialkylamino oder für jeweils gegebenenfalls substituiertes Aryl, Aryloxy oder Arylamino steht,
Ar für jeweils gegebenenfalls substituiertes Phenyl oder Pyridyl steht und
n für eine Zahl 1, 2, 3 oder 4 steht.

2. Verbindungen der allgemeinen Formel (I) gemäß Anspruch 1, worin
$R^1$ für Wasserstoff oder Nitro steht,
$R^2$ für Wasserstoff, für geradkettiges oder verzweigtes Alkyl mit 1 bis 12 Kohlenstoffatomen oder für einen Rest $-\underset{\overset{\|}{O}}{C}-A^1-(O-A^2)_n-Y$ steht,
worin
$A^1$ und $A^2$ unabhängig voneinander jeweils für einen jeweils geradkettigen oder verzweigten, zweifach verknüpften Alkylenrest mit 1 bis 6 Kohlenstoffatomen stehen,

Y für Halogen, Hydroxy, für geradkettiges oder verzweigtes, gegebenenfalls einfach oder mehrfach, gleich oder verschieden durch Halogen, Cyano oder Nitro substituiertes Alkoxy mit 1 bis 6 Kohlenstoffatomen oder für gegebenenfalls einfach oder mehrfach, gleich oder verschieden durch Halogen, Cyano, Nitro, jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 4 Kohlenstoffatomen und gegebenenfalls 1 bis 9 gleichen oder verschiedenen Halogenatomen substituiertes Phenyloxy oder für einen Rest $-O-\underset{\underset{O}{\|}}{C}-R^3$ steht, wobei

$R^3$ für Wasserstoff, Amino, für jeweils geradkettiges oder verzweigtes Alkyl, Alkoxy, Alkylamino oder Dialkylamino mit jeweils 1 bis 6 Kohlenstoffatomen in den einzelnen Alkylteilen oder für jeweils gegebenenfalls einfach oder mehrfach, gleich oder verschieden durch Halogen, Cyano, Nitro, jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 4 Kohlenstoffatomen und gegebenenfalls 1 bis 9 gleichen oder verschiedenen Halogenatomen substituiertes Phenyl, Phenoxy oder Phenylamino steht,

Ar für jeweils gegebenenfalls einfach oder mehrfach gleich oder verschieden durch Halogen, Cyano, Nitro, jeweils geradkettiges oder verzweigtes Alkyl, Alkoxy oder Alkoxycarbonyl mit jeweils 1 bis 4 Kohlenstoffatomen in den Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder einen Rest $-S(O)_m-R^4$, substituiertes Phenyl, 2-Pyridyl, 3-Pyridyl oder 4-Pyridyl steht,

wobei

$R^4$ für Amino, für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht,

m für eine Zahl 0, 1 oder 2 steht und

n für eine Zahl 1, 2, 3 oder 4 steht.

3. Verbindungen der allgemeinen Formel (I) gemäß Anspruch 1, bei welchen

$R^1$ für Wasserstoff oder Nitro steht,

$R^2$ für Wasserstoff, für geradkettiges oder verzweigtes Alkyl mit 1 bis 8 Kohlenstoffatomen oder für einen Rest $-\underset{\underset{O}{\|}}{C}-A^1-(O-A^2)-Y$ steht,

$A^1$ und $A^2$ unabhängig voneinander jeweils für einen jeweils geradkettigen oder verzweigten zweifach verknüpften Alkylenrest mit 1 bis 4 Kohlenstoffatomen stehen,

Y für Fluor, Chlor, Brom, Iod, Hydroxy, geradkettiges oder verzweigtes Alkoxy mit 1 bis 4 Kohlenstoffatomen oder für gegebenenfalls ein-bis dreifach, gleich oder verschieden durch Fluor, Chlor, Brom, Cyano, Nitro, Methyl, Ethyl, n-oder i-Propyl, n-, i-, s-oder t-Butyl, Methoxy, Ethoxy, Methylthio, Trifluormethyl, Trifluormethoxy, Trifluormethylthio, Methoxycarbonyl oder Ethoxycarbonyl substituiertes Phenyloxy steht, oder für einen Rest $-O-\underset{\underset{O}{\|}}{C}-R^3$ steht,

$R^3$ für Wasserstoff, Amino, für jeweils geradkettiges oder verzweigtes Alkyl, Alkoxy, Alkylamino oder Dialkylamino mit jeweils 1 bis 4 Kohlenstoffatomen in den einzelnen Alkylteilen oder für jeweils gegebenenfalls ein-bis dreifach, gleich oder verschieden durch Fluor, Chlor, Brom, Cyano, Nitro, Methyl, Ethyl, n-oder i-Propyl, n-, i-, s-oder t-Butyl, Methoxy, Ethoxy, Methylthio, Trifluormethyl, Trifluormethoxy, Trifluormethylthio, Methoxycarbonyl oder Ethoxycarbonyl, substituiertes Phenyl, Phenoxy oder Phenylamino steht,

Ar für gegebenenfalls ein-bis fünffach, gleich oder verschieden durch Cyano, Nitro, Fluor, Chlor, Brom, Iod, Methyl, Ethyl, n-oder i-Propyl, n-, i-, s-oder t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder einen Rest $-S(O)_m-R^4$ substituiertes Phenyl oder für jeweils gegebenenfalls ein-bis vierfach, gleich oder verschieden durch die gleichen Substituenten wie bei Phenyl substituiertes 2-Pyridyl, 3-Pyridyl oder 4-Pyridyl steht,

wobei

$R^4$ für Amino, Methylamino, Ethylamino, Dimethylamino, Diethylamino, Fluordichlormethyl, Difluormethyl, Tetrafluorethyl, Trichlorethyl, Trifluormethyl, Difluorchlormethyl, Methyl oder Ethyl steht,

m für eine Zahl 0, 1 oder 2 steht und

n für eine Zahl 1, 2, 3 oder 4 steht.

4. Verbindungen der allgemeinen Formel (I), gemäß Anspruch 1, bei welchen
$R^1$ für Nitro steht,
$R^2$ für Wasserstoff, Methyl, Ethyl, n-oder i-Propyl, n-, i-, s-oder t-Butyl oder für einen Rest $-\underset{\underset{O}{\|}}{C}-A^1-(O-A^2)_n-Y$ steht,

$A^1$ und $A^2$ unabhängig voneinander jeweils für ein Brückenglied der Formel $-CH_2-$, $-CH_2-CH_2-$;

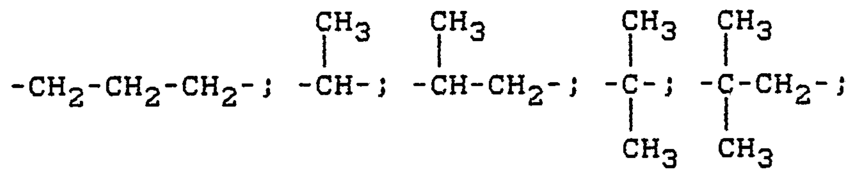

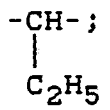

oder

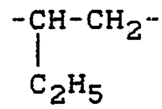

stehen,

Y für Fluor, Chlor, Brom, Methoxy, Ethoxy, n-oder i-Propoxy, n-oder i-Butoxy oder Hydroxy steht,
Ar für gegebenenfalls ein-bis fünfach, gleich oder verschieden durch Cyano, Nitro, Fluor, Chlor, Brom, Iod, Methyl, Ethyl, n-oder i-Propyl, n-, i-, s-oder t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder einen Rest $-S(O)_m-R^4$ substituiertes Phenyl oder für gegebenenfalls ein-bis vierfach gleich oder verschieden durch die gleichen Substituenten wie bei Phenyl substituiertes 2-Pyridyl steht,
wobei
$R^4$ für Amino, Methylamino, Ethylamino, Dimethylamino, Diethylamino, Fluordichlormethyl, Difluormethyl, Tetrafluorethyl, Trichlorethyl, Trifluormethyl, Difluorchlormethyl, Methyl oder Ethyl steht,
m für eine Zahl 0, 1 oder 2 steht und
n für eine Zahl 1 oder 2 steht.

5. Verfahren zur Herstellung von substituierten 1-Arylpyrazolen der allgemeinen Formel (I),

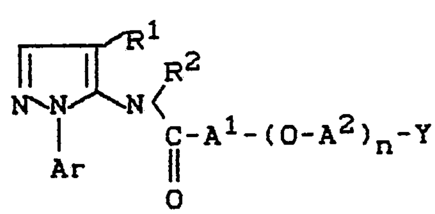

(I)

in welcher
$R^1$ für Wasserstoff oder Nitro steht,
$R^2$ für Wasserstoff, Alkyl oder für einen Rest $-\underset{\underset{O}{\|}}{C}-A^1-(O-A^2)_n-Y$ steht,

$A^1$ und $A^2$ unabhängig voneinander jeweils für einen zweifach verknüpften Alkylenrest stehen,
Y für Halogen, Hydroxy, für gegebenenfalls substituiertes Alkoxy, für gegebenenfalls substituiertes Aryloxy oder für einen Rest $-O-\underset{\underset{O}{\|}}{C}-R^3$ steht,

$R^3$ für Wasserstoff, Alkyl, Alkoxy, Amino, Alkylamino, Dialkylamino oder für jeweils gegebenenfalls substituiertes Aryl, Aryloxy oder Arylamino steht,
Ar für jeweils gegebenenfalls substituiertes Phenyl oder Pyridyl steht und
n für eine Zahl 1, 2, 3 oder 4 steht,
dadurch gekennzeichnet, daß man, um

(a) substituierte 1-Arylpyrazole der Formel (Ia)

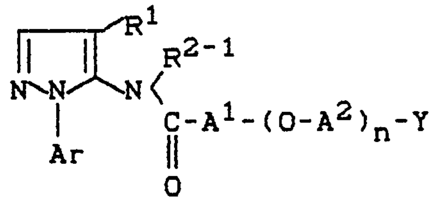

(Ia)

zu erhalten,
in welcher
$R^1$, $A^1$, $A^2$, Y, Ar und n die oben angegebene Bedeutung haben und
$R^{2-1}$ für Wasserstoff oder für einen Rest $-\underset{\underset{O}{\|}}{C}-A^2-(O-A^1)_n-Y$ steht,

5-Amino-1-aryl-pyrazole der allgemeinen Formel (II),

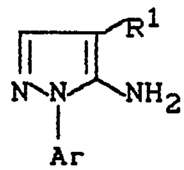

(II) .

in welcher
$R^1$ und Ar die oben angegebene Bedeutung haben,
mit Acylierungsmitteln der allgemeinen Formel (III),

$Hal^1-\underset{\underset{O}{\|}}{C}-A^1-(O-A^2)_n-Y$ (III)

in welcher
$Hal^1$ für Halogen steht und
$A^1$, $A^2$, Y und n die oben angegebene Bedeutung haben,
gegebenenfalls in Gegenwart eines Verdünnungsmittels, gegebenenfalls in Gegenwart eines Säurebindemittels und gegebenenfalls in Gegenwart eines Katalysators umsetzt;

(b) oder daß man, um substituierte 1-Arylpyrazole der allgemeinen Formel (Ib),

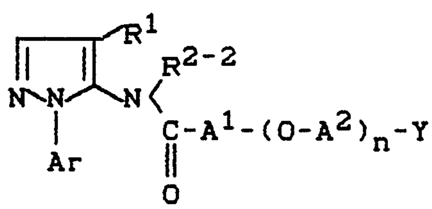

(Ib)

zu erhalten,
in welcher
$R^{2-2}$ für Alkyl steht und
$R^1$, $A^1$, $A^2$, Y, Ar und ndie oben angegebene Bedeutung haben,
substituierte 1-Arylpyrazole der allgemeinen Formel (Ic),

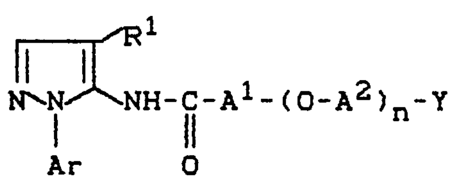

(Ic)

in welcher

$R^1$, $A^1$, $A^2$, Y, Ar und n die oben angegebene Bedeutung haben,
mit Alkylierungsmitteln der allgemeinen Formel (IV),

$R^{2-2}$-E (IV)

in welcher
$R^{2-2}$ für Alkyl steht und
E für eine elektronenanziehende Abgangsgruppe steht,
gegebenenfalls in Gegenwart eines Verdünnungsmittels und gegebenenfalls in Gegenwart eines Säurebindemittels sowie gegebenenfalls in Gegenwart eines Katalysators umsetzt;

(c) oder daß man, um 4-Nitro-1-aryl-pyrazole der allgemeinen Formel (Id),

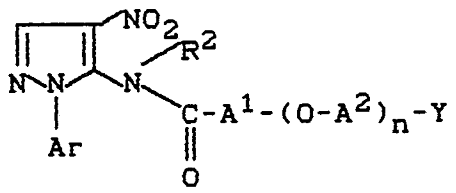

(Id)

zu erhalten,
in welcher
$R^2$, $A^1$, $A^2$, Y, Ar und n die oben angegebene Bedeutung haben,
substituierte 1-Arylpyrazole der allgemeinen Formel (Ie),

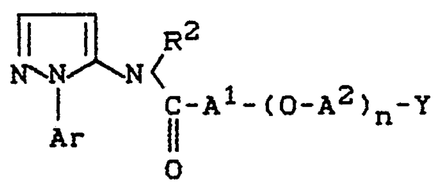

(Ie)

in welcher
$R^2$, $A^1$, $A^2$, Y, Ar und n die oben angegebene Bedeutung haben,
mit einem Nitrierungsmittel gegebenenfalls in Gegenwart eines Verdünnungsmittels und gegebenenfalls in Gegenwart eines Katalysators oder Reaktionshilfmittels umsetzt;

(d) oder daß man, um substituierte 1-Arylpyrazole der allgemeinen Formel (If)

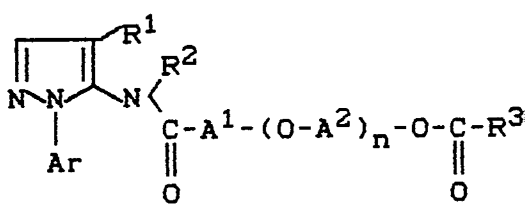

(If)

zu erhalten,
in welcher
$R^1$, $R^2$, $R^3$, $A^1$, $A^2$, Ar und n die oben angegebene Bedeutung haben,
1-Arylpyrazole der allgemeinen Formel (Ig),

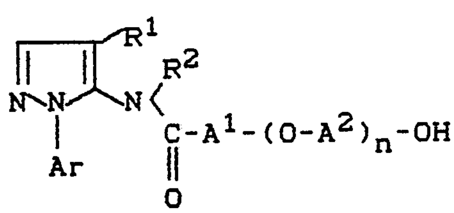

(Ig)

in welcher

$R^1$, $R^2$, $A^1$, $A^2$, Ar und n die oben angegebene Bedeutung haben,
mit Acylierungsmitteln der allgemeinen Formel (VI),

$$R^3-\underset{\underset{O}{\|}}{C}-Hal^2 \qquad (VI)$$

in welcher
$Hal^2$ für Halogen steht und
$R^3$ die oben angegebene Bedeutung hat,
gegebenenfalls in Gegenwart eines Verdünnungsmittels, gegebenenfalls in Gegenwart eines Säurebindemittels und gegebenenfalls in Gegenwart eines Acylierungskatalysators umsetzt.

6. Herbizide und pflanzenwuchsregulierende Mittel, gekennzeichnet durch einen Gehalt an mindestens einem 1-Arylpyrazol der allgemeinen Formel (I) gemäß den Ansprüchen 1 bis 5.

7. Verfahren zur Bekämpfung von Unkräutern, dadurch gekennzeichnet, daß man 1-Arylpyrazole der allgemeinen Formel (I) gemäß den Ansprüchen 1 bis 5 auf die Unkräuter und/oder ihren Lebensraum einwirken läßt.

8. Verwendung von 1-Arylpyrazolen der allgemeinen Formel (I) gemäß den Ansprüchen 1 bis 5 zur Bekämpfung von Unkräutern und als Pflanzenwuchsregulatoren.

9. Verfahren zur Herstellung von herbiziden und pflanzenwuchsregulierenden Mitteln, dadurch gekennzeichnet, daß man 1-Arylpyrazole der allgemeinen Formel (I) gemäß den Ansprüchen 1 bis 5 mit Streckmitteln und/oder oberflächenaktiven Substanzen vermischt.

Europäisches Patentamt

# EUROPÄISCHER RECHERCHENBERICHT

Nummer der Anmeldung

EP 88102979.7

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A,P | DE - A1 - 3 618 717 (BAYER AG)<br>* Zusammenfassung *<br>-- | 1,5,6-9 | C 07 D 231/40<br>C 07 D 401/12<br>A 01 N 43/56 |
| A,P | DE - A1 - 3 600 950 (BAYER AG)<br>* Zusammenfassung *<br>-- | 1,5,6-9 | |
| A,P | CHEMICAL ABSTRACTS, Band 107, Nr. 23, 7. Dezember 1987, Columbus, Ohio, USA<br>TOKUYAMA SODA CO. "Preparation of α-[(pyridyloxy)alkyl]-N--pyrazolylacetamides as herbicides and antimicrobiuls"<br>Seite 591, Spalte 2, Zusammenfassung-Nr. 217 623t<br>& Jpn. Kokai Tokkyo Koho JP 62 153 283 [87 153 283]<br>-- | 1,5,6-9 | |
| | | | RECHERCHIERTE SACHGEBIETE (Int. Cl.4) |
| A,P | CHEMICAL ABSTRACTS, Band 107, Nr. 21, 23. November 1987, Columbus, Ohio, USA<br>TOKUYAMA SODA CO. "Preparation of α-(phenoxyalkyl)-N-pyrazolylacetamides as bactericides, herbicides, and fungicides."<br>Seite 765, Spalte 1, Zusammenfassung-Nr. 198 317c<br>& Jpn. Kokai Tokkyo Koho JP 62 153 273 [87 153 273]<br>---- | 1,5,6-9 | C 07 D 231/00<br>C 07 D 401/00 |

Der vorliegende Recherchenbericht wurde für alle Patentansprüche erstellt.

| Recherchenort | Abschlußdatum der Recherche | Prüfer |
|---|---|---|
| WIEN | 13-05-1988 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82